# EUROPEAN PATENT SPECIFICATION

(11) **EP 1 904 171 B1**
(45) Date of publication and mention of the grant of the patent: **19.10.2016**
(21) Application number: 06750542.0
(22) Date of filing: 17.04.2006
(51) Int. Cl.: A61N 1/372, A61N 1/37, A61B 5/00, A61B 5/04, A61B 5/044, A61N 1/362, A61N 1/36, A61N 1/365

(54) **SYSTEM FOR SIMULTANEOUSLY PRESENTING CARDIAC AND NEURAL SIGNALS**
SYSTEM ZUR SIMULTANEN DARSTELLUNG VON HERZ- UND NERVENSIGNALEN
SYSTEME POUR PRESENTER SIMULTANEMENT DES SIGNAUX CARDIAQUES ET NEURONAUX

(30) Priority: 25.04.2005 US 114246
(43) Date of publication of application: 02.04.2008
(73) Proprietor: CARDIAC PACEMAKERS, INC., St. Paul, Minnesota 55164-5798 (US)
(72) Inventor: LIBBUS, Imad, St. Paul, Minnesota 55105 (US); KRAMER, Andrew P., Marine on St. Croix, Minnesota 55047 (US); LINDER, William J., Golden Valley, Minnesota 55422 (US); STAHMANN, Jeffrey E., Ramsey, Minnesota 55303 (US)
(74) Representative: Pfenning, Meinig & Partner mbB
(86) International application number: PCT/US2006/014535
(87) International publication number: WO 2006/115899

(56) References cited:
- EP-A- 0 770 409
- WO-A-2004/036372
- US-A- 4 809 697
- US-A1- 2004 243 188
- US-B1- 6 535 763

## Description

### FIELD OF THE INVENTION

This document generally relates to medical devices and particularly to a system including a user interface that simultaneously presents cardiac and neural signals.

### BACKGROUND

The heart is the center of a person's circulatory system. The left portions of the heart draw oxygenated blood from the lungs and pump it to the organs of the body to provide the organs with their metabolic needs for oxygen. The right portions of the heart draw deoxygenated blood from the body organs and pump it to the lungs where the blood gets oxygenated. These pumping functions are accomplished by cyclic contractions of the myocardium (heart muscles). In a normal heart, the sinoatrial node generates electrical impulses called action potentials. The electrical impulses propagate through an electrical conduction system to various regions of the heart to excite the myocardial tissue of these regions. Coordinated delays in the propagations of the action potentials in a normal electrical conduction system cause the various portions of the heart to contract in synchrony to result in efficient pumping functions indicated by a normal hemodynamic performance. A blocked or otherwise abnormal electrical conduction system and/or deteriorated myocardial tissue result in an impaired hemodynamic performance, including a diminished blood supply to the heart and the rest of the body.

The hemodynamic performance is modulated by neural signals in portions of the autonomic nervous system. For example, the myocardium is innervated with sympathetic and parasympathetic nerves. Activities in these nerves, including artificially applied electrical stimuli, modulate cardiac functions and hemodynamic performance. Direct electrical stimulation of parasympathetic nerves can activate the baroreflex, inducing a reduction of sympathetic nerve activity and reducing blood pressure by decreasing vascular resistance. Sympathetic inhibition, as well as parasympathetic activation, has been associated with reduced arrhythmia vulnerability following a myocardial infarction, presumably by increasing collateral perfusion of the acutely ischemic myocardium and decreasing myocardial damage. Modulation of the sympathetic and parasympathetic nervous system with neural stimulation has been shown to have positive clinical benefits, such as protecting the myocardium from further remodeling and predisposition to fatal arrhythmias following a myocardial infarction.

The effects of a neural stimulation therapy in cardiac functions and hemodynamic performance are indicated by cardiac signals indicative of the cardiac functions and hemodynamic performance. Thus, to guide the neural stimulation therapy, there is a need to provide a means for observing and analyzing the effects of neural events including intrinsic neural activities and artificial neural stimuli in the cardiac signals. Additionally, electrical stimulation therapies delivered to the heart, such as pacing and defibrillation therapies, have been developed and applied to treat various cardiac disorders including arrhythmias and heart failure and to control myocardial remodeling. When combined cardiac and neural stimulation therapies are applied, there is a need to provide a means for observing and analyzing the effects of both therapies in cardiac and/or neural signals.

EP0770409A2 relates to an implantable atrial defibrillator and to a system having multiple channel electrogram telemetry. The defibrillator includes a cardioverting device responsive to at least one of a first and a second sensing device for applying cardioverting electrical energy to the heart, and a formatting device for formatting the first and second electrogram data for combined transmission. A transmitting device is used for transmitting the formatted first and second electrogram data to a non-implanlable external receiver for simultaneous display of the first and second electrogram data. If atrial fibrillation is detected by an atrial fibrillation detector, a charge delivery control causes the charge and storage capacitor circuit to charge the storage capacitor to a preselected peak voltage. When the capacitor is charged, the atrial fibrillation detector determines if the atria of heart are still in fibrillation. In doing 50, the atrial defibrillator will perform another eight second data acquisition.

EP0770409A2 does not disclose: a telemetry circuit configured to receive data including neural event markers indicative of neural stimulation periods; a presentation controller adapted to produce neural signals indicative of autonomic nerve traffic and indicative of neural stimulation periods for visual presentation, and further adapted to temporally align the neural signals with one or more cardiac signals indicative of cardiac depolarizations; and a presentation device adapted to simultaneously present the temporally aligned cardiac and neural signals to allow for observation of relationships between the cardiac and neural activities.

### SUMMARY

The invention is defined in the appended claims.

A presentation device such as a display screen or a printer provides for simultaneous presentation of temporally aligned cardiac and neural signals. At least one cardiac signal in the form of a cardiac signal trace or cardiac event
markers and at least one neural signal in the form of a neural signal trace or neural event markers are simultaneously presented. The cardiac signal indicates sensed cardiac electrical activities and/or cardiac stimulation pulse deliveries. The neural signal indicates sensed neural electrical activities and/or neural stimulation pulse deliveries.

In one embodiment, a system communicating with one or more implantable medical devices includes a telemetry circuit, an external control circuit, and a presentation device. The telemetry circuit receives data representative of cardiac and neural activities from the one or more implantable medical devices. The external control circuit includes a presentation controller that produces and temporally aligns one or more cardiac signals and one or more neural signals for visual presentation based on the received data. The presentation device simultaneously presents the temporally aligned one or more cardiac signals and one or more neural signals.

In one embodiment, a medical device system includes an implantable system and an external system. The implantable system includes a cardiac sensing circuit, a cardiac stimulation circuit, a neural sensing circuit, a neural stimulation circuit, an implant control circuit, and an implant telemetry circuit. The cardiac sensing circuit senses at least one cardiac signal indicative of cardiac electrical activities. The cardiac stimulation circuit delivers cardiac stimulation pulses. The neural sensing circuit senses at least one neural signal indicative of neural electrical activities. The neural stimulation circuit delivers neural stimulation pulses. The implant control circuit produces data representative of the cardiac electrical activities, the delivered cardiac stimulation pulses, the neural electrical activities, and the delivered neural stimulation pulses. The implant telemetry circuit transmits the data. The external system is communicatively coupled to the implant system via telemetry and includes an external telemetry circuit, an external control circuit, and a presentation device. The external telemetry circuit receives the data transmitted from the implant telemetry circuit. The external control circuit includes a presentation controller that produces and temporally aligns one or more cardiac signals and one or more neural signals. The one or more cardiac signals represent at least one of the cardiac electrical activities and the delivered cardiac stimulation pulses. The one or more neural signals represent at least one of the neural electrical activities and the delivered neural stimulation pulses. The presentation device simultaneously presents the temporally aligned one or more cardiac signals and one or more neural signals.

In one embodiment, a method for presenting cardiac and neural activities is provided. Data representative of cardiac and neural activities are received from one or more implantable medical devices. Cardiac and neural signals are produced for presentation based in the received data. The cardiac and neural signals are temporally aligned. The temporally aligned cardiac and neural signals are presented.

This Summary is an overview of some of the teachings of the present application and not intended to be an exclusive or exhaustive treatment of the present subject matter. Further details about the present subject matter are found in the detailed description and appended claims. Other aspects of the invention will be apparent to persons skilled in the art upon reading and understanding the following detailed description and viewing the drawings that form a part thereof. The scope of the present invention is defined by the appended claims and their legal equivalents.

### BRIEF DESCRIPTION OF THE DRAWINGS

In the drawings, which are not necessarily drawn to scale, like numerals describe similar components throughout the several views. The drawings illustrate generally, by way of example, various embodiments discussed in the present document.
FIG. 1 is an illustration of an embodiment of a cardiac and neural stimulation system including an implantable system and an external system and portions of an environment in which the cardiac and neural stimulation system is used.
FIG. 2 is a block diagram illustrating an embodiment of a circuit of the implantable system.
FIG. 3 is a block diagram illustrating an embodiment of a signal processing circuit of the cardiac and neural stimulation system.
FIG. 4 is a block diagram illustrating an embodiment of a user interface of the cardiac and neural stimulation system.
FIG. 5 is a flow chart illustrating an embodiment of a method for simultaneously presenting cardiac and neural signals.
FIGS. 6A-E are each an illustration of an exemplary embodiment of a display window presenting at least a cardiac signal trace and neural event markers.
FIGS. 7A-C are each an illustration of an exemplary embodiment of a display window presenting at least a neural signal trace and cardiac event markers.
FIG. 8 is an illustration of an exemplary embodiment of a display window presenting at least a cardiac signal trace and a neural signal trace.
FIG. 9 is an illustration of an exemplary embodiment of a display window presenting at least cardiac event markers and neural event markers.
FIG. 10 is an illustration of an exemplary embodiment of a display window presenting physiologic parameters in addition to the cardiac and neural signals.
FIGS. 11A and 11B are illustrations of neural mechanisms for peripheral vascular control.
FIGS. 12A-C are illustration of a heart.
FIG. 13 is an illustration of baroreceptors and afferent nerves in the area of the carotid sinuses and aortic arch.
FIG. 14 is an illustration of baroreceptors in and around the pulmonary artery.
FIG. 15 is an illustration of baroreceptor fields in the aortic arch, the ligamentum arteriosum and the trunk of the pulmonary artery.
FIG. 16 is an illustration of an example of a neural response after perturbing a physiologic system.
FIG. 17 is an illustration of a specific embodiment of the cardiac and neural stimulation system.
FIG. 18 is an illustration of another specific embodiment of the cardiac and neural stimulation system.
FIG. 19 is a block diagram illustrating an embodiment of a circuit of the cardiac and neural stimulation system that provides for the simultaneous presentation of cardiac and neural signals.
FIG. 20 is a block diagram illustrating a specific embodiment of the external system.

### DETAILED DESCRIPTION

In the following detailed description, reference is made to the accompanying drawings which form a part hereof, and in which is shown by way of illustration specific embodiments in which the invention may be practiced. These embodiments are described in sufficient detail to enable those skilled in the art to practice the invention, and it is to be understood that the embodiments may be combined, or that other embodiments may be utilized and that structural, logical and electrical changes may be made without departing from the spirit and scope of the present invention. The following detailed description provides examples, and the scope of the present invention is defined by the appended claims and their legal equivalents.

It should be noted that references to "an", "one", or "various" embodiments in this disclosure are not necessarily to the same embodiment, and such references contemplate more than one embodiment.

This document discusses a cardiac and neural stimulation system that includes a presentation device such as a display screen or a printer for simultaneously presenting cardiac and neural signals. The cardiac and neural signals are temporally aligned by the time at which they are sensed. The presentation device presents at least a cardiac signal trace or cardiac event markers and at least a neural signal trace or neural event markers. The cardiac signal indicates sensed cardiac electrical events and deliveries of cardiac electrical stimulation pulses such as pacing or defibrillation pulses. The neural signal indicates sensed neural electrical events and deliveries of neural electrical stimulation pulses. The simultaneous presentation of the temporally aligned cardiac and neural signals allows for observation of analysis of relationships between cardiac events and neural events, such as effects of neural stimulation in neural electrical activities and cardiac rhythms.

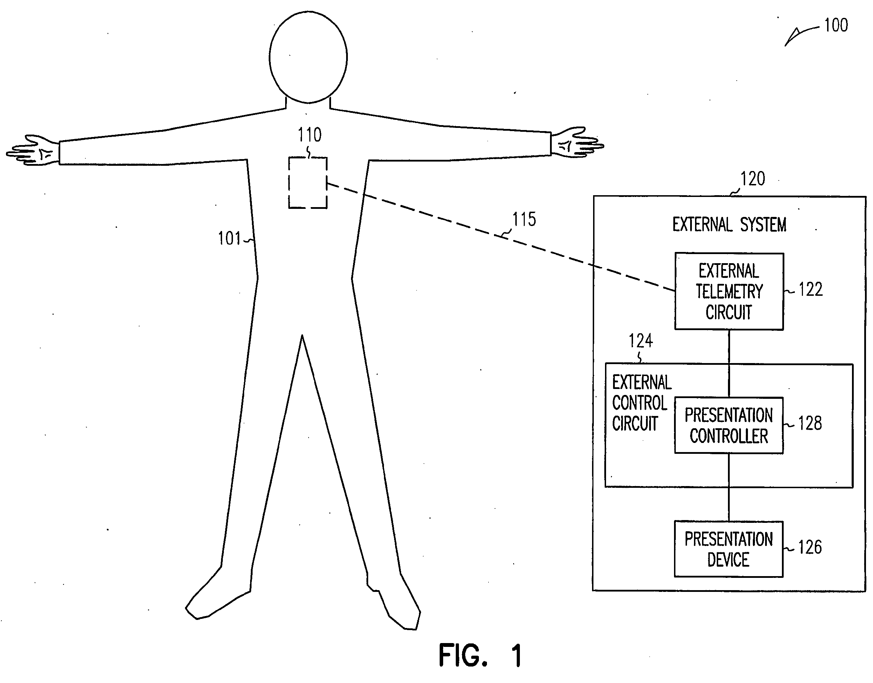

FIG. 1 is an illustration of an embodiment of a cardiac and neural stimulation system 100 and portions of an environment in which system 100 is used. System 100 includes an implantable system 110, an external system 120, and a telemetry link 115.

Implantable system 110 includes one or more implantable medical devices. After being implanted in a patient's body 101, implantable system 110 senses cardiac and neural signals and delivers electrical stimulation pulses to the heart and/or one or more nerves that regulate cardiac functions and hemodynamic performance. Implantable system 110 produces data representative of cardiac and neural activities and transmits the data to external system 120. The data representative of cardiac activities include one or more sensed cardiac signals, such as electrograms, and/or cardiac event markers representative of detected cardiac events such as detected depolarizations and cardiac stimulation pulse deliveries. The data representative of neural activities include one or more sensed neural signals and/or neural event markers representative of detected neural events and neural stimulation pulse deliveries.

External system 120 receives and processes the data transmitted from implantable system 110 and controls the operation of implantable system 110. In the illustrated embodiment, external system 120 includes an external telemetry circuit 122, an external control circuit 124, and a presentation device 126. Telemetry circuit 122 receives the data representative of the cardiac and neural activities from implantable system 110. External control circuit 124 processes the data received by telemetry circuit 122 and includes a presentation controller 128. Presentation controller 128 produces and temporally aligns selected type cardiac and neural signals for visual presentation. Such signals for visual presentation include one or more types of signal traces and one or more types of event markers. Presentation device 126 simultaneously presents the temporally aligned selected type cardiac and neural signals. In one embodiment, external control circuit 124 further produces physiologic parameters or signals based on the data representative of the cardiac and neural activities. Such physiologic parameters or signals indicate cardiac and/or hemodynamic response to cardiac and/or neural stimulation. Presentation device 126 further presents one or more selected type physiologic parameters or signals simultaneously with the temporally aligned selected type cardiac and neural signals.

Telemetry link 115 provides for communication between implantable system 110 and external system 120. In one embodiment, telemetry link 115 is an inductive telemetry link. In an alternative embodiment, telemetry link 115 is a far-field radio-frequency telemetry link. The communication includes data transmission from implantable system 110 to external system 120, including, for example, transmitting the data representative of the cardiac and neural activities in real time, extracting the data representative of the cardiac and neural activities stored in implantable system 110, and extracting data indicating an operational status of implantable system 110 (e.g., battery status and lead impedance). The communication also includes data transmission from external system 120 to implantable system 110, including, for example, programming implantable system 110 to produce the data representative of the cardiac and neural activities, programming implantable system 110 to perform at least one self-diagnostic test (such as for a device operational status), and programming implantable system 110 to deliver at least one of the cardiac and neural stimulation therapies.

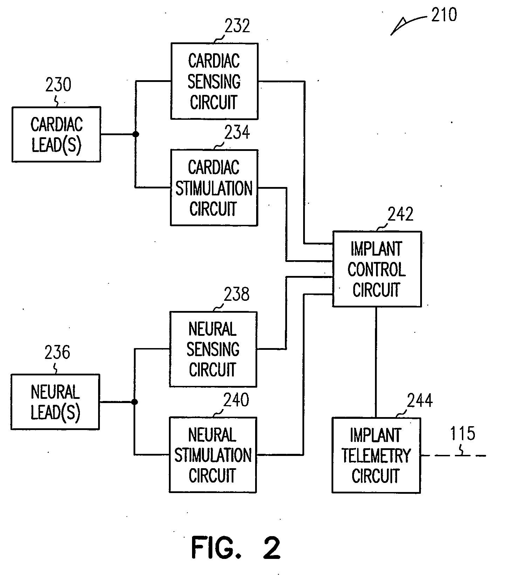

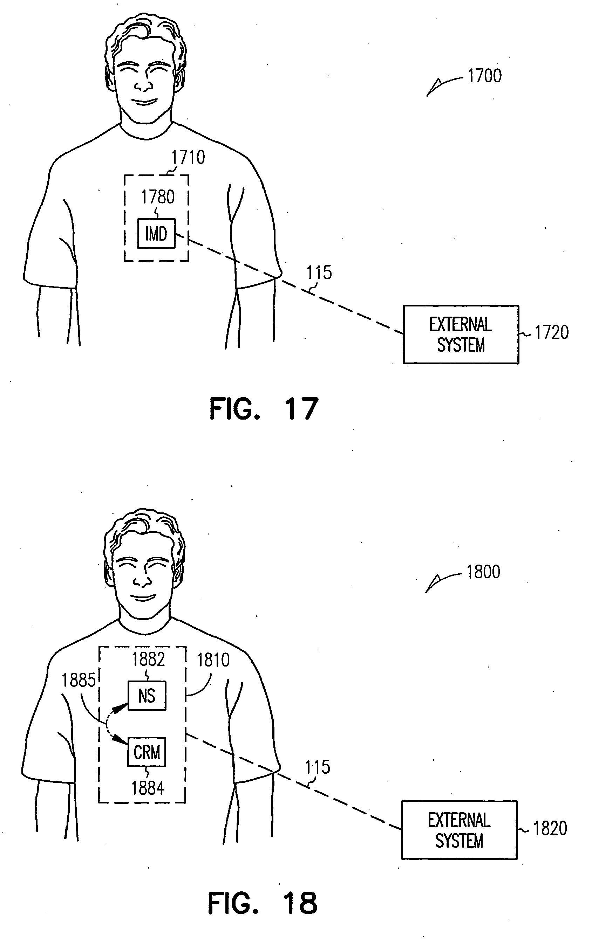

FIG. 2 is a block diagram illustrating an embodiment of a circuit of implantable system 210, which is a specific embodiment of implantable system 110. In various embodiments, the circuit is included on a single implantable device or distributed in two or more implantable devices, as further discussed below with reference to FIGS. 17 and 18. Implantable system 210 includes one or more cardiac leads 230, a cardiac sensing circuit 232, a cardiac stimulation circuit 234, one or more neural leads 236, a neural sensing circuit 238, a neural stimulation circuit 240, an implant control circuit 242, and an implant telemetry circuit 244.

Cardiac lead(s) 230 are cardiac sensing/stimulation leads each including one or more endocardial or epicardial electrodes for sensing one or more cardiac signals indicative of cardiac electrical activities and/or delivering cardiac stimulation pulses. Examples of such cardiac leads include pacing and defibrillation leads each include at least one electrode for sensing an electrogram. In various embodiments, electrodes are configured to be placed in, near, or over the right atrium (RA), left atrium (LA), right ventricle (RV), and/or left ventricle (LV) to sense electrograms indicative of depolarizations in these chambers. Cardiac sensing circuit 232 senses one or more cardiac signals through cardiac lead(s) 230. Cardiac stimulation circuit 234 delivers cardiac stimulation pulses through cardiac lead(s) 230.

Neural lead(s) 236 are neural sensing/stimulation leads each including one or more electrodes for sensing one or more neural signals indicative of neural electrical activities and/or delivering neural stimulation pulses. Examples of such neural leads include an expandable stimulation lead having an electrode for placement in a pulmonary artery in a proximity of a high concentration of baroreceptors, a transvascular lead having an electrode for placement proximal to one of the cardiac fat pads, an epicardial lead having an electrode for placement in the cardiac fat pad, a lead having a cuff electrode for placement around an aortic, carotid, or vagus nerve, and an intravascularly fed lead having an electrode for placement proximal to the aortic, carotid, or vagus nerve for transvascularly stimulating that nerve. Neural sensing circuit 238 senses one or more neural signals through neural lead(s) 236. Neural stimulation circuit 240 delivers neural stimulation pulses through neural lead(s) 236.

Implant control circuit 242 controls the operation of implantable system 210 and produces the data representative of the cardiac and neural activities, including the one or more sensed cardiac signals, the delivered cardiac stimulation pulses, the one or more sensed neural signals, and the delivered neural stimulation pulses. Implant telemetry circuit 244 transmit the data to external system 120 via telemetry link 115. In one embodiment, implant control circuit 242 time stamps the cardiac and neural activities, including the one or more sensed cardiac signals, the delivered cardiac stimulation pulses, the one or more sensed neural signals, and the delivered neural stimulation pulses. The data representative of the cardiac and neural activities are then transmitted over telemetry link serially or by multiplexing. External system 120 reconstructs the sequence and timing of the cardiac and neural activities using the time stamps to provide for the presentation of the temporally aligned cardiac and neural signals. In one embodiment, implant control circuit 242 time stamps each of predetermined type events selected from the detected cardiac events, detected neural events, delivered cardiac stimulation pulses, and delivered neural stimulation pulses. In another embodiment, implant control circuit 242 stamps the start and end times for each of the predetermined type events. In another embodiment, implant control circuit 242 stamps the start time and the duration for each of the predetermined type events. In an alternative embodiment, implant control circuit 242 produces periodic timing interval markers to provide for a common timing reference for all the cardiac and neural activities.

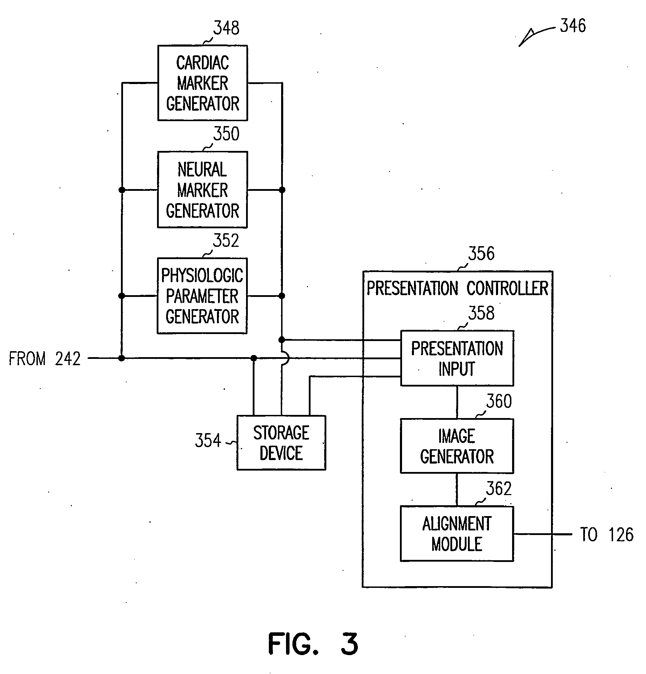

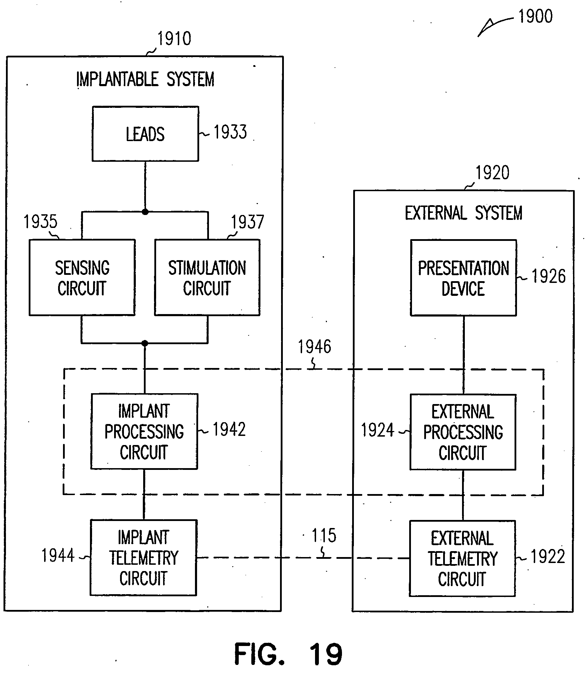

FIG. 3 is a block diagram illustrating an embodiment of a signal processing circuit 346 of system 100. Signal processing circuit 346 produces the signals for visual presentation by presentation device 126. Signal processing circuit 346 includes a cardiac marker generator 348, a neural marker generator 350, a physiologic parameter generator 352, a storage device 354, and a presentation controller 356. In various embodiments, signal processing circuit 346 is distributed as part of implant control circuit 242 and external control circuit 124, as further discussed below with reference to FIG. 19.

Cardiac marker generator 348 produces cardiac event markers indicative of predetermined type cardiac events. The cardiac event markers include cardiac stimulation markers each indicative of a delivery of a cardiac stimulation pulse and cardiac sense markers each indicative of an intrinsic cardiac electrical event. Each cardiac event marker is a distinctive symbol associated of a particular type cardiac event and is time stamped, using timing information provided by cardiac sensing circuit 232, to indicate the time of occurrence or detection of that cardiac event.

Neural marker generator 350 produces neural event markers indicative of predetermined type neural events. The neural event markers include neural stimulation markers each indicative of a delivery of a neural stimulation pulse and neural sense markers each indicative of an intrinsic neural electrical event. Each neural event marker is a distinctive symbol indicative of a particular type neural event and is time stamped, using timing information provided by neural sensing circuit 238, to indicate the time of occurrence or detection of that neural event. In one embodiment, the neural stimulation markers include markers each representative of a neural stimulation period during which a burst of the neural stimulation pulses is delivered.

Physiologic parameter generator 352 derives one or more physiologic parameters from the data representative of the cardiac and neural activities. In one embodiment, physiologic parameter generator 352 includes a heart rate generator to dynamically measure a heart rate. In a further embodiment, physiologic parameter generator 352 produces a heart rate signal that represents the measured heart rate and shows change in the heart rate over time. In another embodiment, physiologic parameter generator 352 includes a heart rate variability (HRV) generator to dynamically calculate an HRV parameter based on the measured heart rate. In a further embodiment, physiologic parameter generator 352 produces an HRV signal to represent the calculated HRV parameter and shows change in the HRV over time. In another embodiment, physiologic parameter generator 352 includes a cardiac interval generator to dynamically measure a predetermined type cardiac interval. Examples of such cardiac interval include cardiac cycle length, atrioventricular interval (AVI); and interventricular interval (IVI). In a further embodiment, physiologic parameter generator 352 produces a cardiac interval signal to represent the measured cardiac interval and shows change in the cardiac interval over time. In another embodiment, physiologic parameter generator 352 includes an amplitude generator to dynamically measure an amplitude associated with a predetermined type cardiac event. In a further embodiment, physiologic parameter generator 352 produces an amplitude signal to represent the measured amplitude of the predetermined type cardiac event and shows change in that amplitude over time. In another embodiment, physiologic parameter generator 352 includes a duration generator to dynamically measure a duration associated with a predetermined type cardiac event. Examples of such cardiac events include P-wave, R-wave, and T-wave. In a further embodiment, physiologic parameter generator 352 produces a duration signal to represent the measured duration of the predetermined type cardiac event and shows change in that duration over time.

Storage device 354 stores data representing some or all of the sensed cardiac and neural signals, the cardiac and neural event markers, and the physiologic parameters and/or signals. When needed, storage device 354 allows for diagnosis or therapy control based on stored data.

Presentation controller 356 controls presentation device 126. Presentation controller 356 includes a presentation input 358, an image generator 360, and an alignment module 362. Presentation input 358 receives some or all of the sensed cardiac and neural signals, the cardiac and neural event markers, and the physiologic parameters and/or signals. In one embodiment, presentation input 358 receives data from implant control circuit 242 for presenting the cardiac and neural signal in real time. In another embodiment, presentation input 358 receives data from storage device 354 for presenting stored cardiac and neural signals for an off-line analysis. Image generator 360 produces visual images for the cardiac and neural signals. Alignment module 362 temporally aligns the visual images of the cardiac and neural signals based on their timing information (such as the time stamps) for simultaneous presentation by presentation device 126. In one embodiment, image generator 360 further produces one or more visual images for the physiologic parameters or signals, and alignment module 362 further temporally aligns the visual image(s) for the physiologic parameters or signals with visual images of the cardiac and/or neural signals for simultaneous presentation by presentation device 126. In one embodiment, presentation controller 356 receives user commands and controls the content of the presentation according to the user commands. Presentation input 358 selectively receives data representative of the sensed cardiac and neural signals, the cardiac and neural event markers, and the physiologic parameters or signals according to the user command. Image generator 360 selectively produces the images for the signals according to the user commands. Alignment module 362 temporally aligns the selectively produced images for simultaneous presentation by presentation device 126.

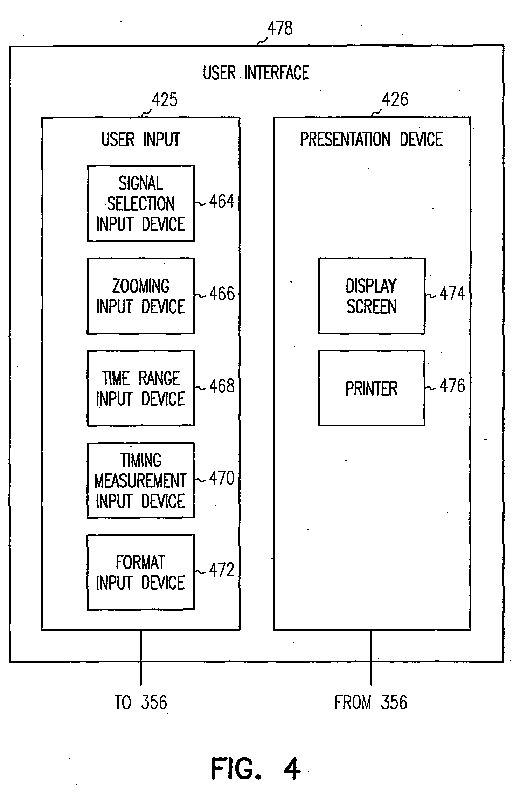

FIG. 4 is a block diagram illustrating an embodiment of a user interface 478 of system 100. User interface 478 is part of external system 120 and includes a user input 425 and presentation device 426.

User input 425 includes a plurality of user input devices to receive user commands controlling the content and the format of the visual presentation of the cardiac and neural signals. Examples of such user input devices include a signal selection input device 464, a zooming input device 466, a time range input device 468, a timing measurement input device 470, and a format input device 472. Signal selection input device 464 receives user commands controlling the content of presentation. The user, such as a physician or other caregiver, is allowed to select at least one type of cardiac signal and at least one type of neural signal for simultaneous presentation by presentation device 126. In one embodiment, the user is further allowed to select at least one type of physiologic parameter or signal for simultaneous presentation with the cardiac and neural signals. Examples of the signals selectable for simultaneous presentation include the one or more cardiac signals sensed by cardiac sensing circuit 232, the one or more neural signals sensed by neural sensing circuit 238, the cardiac event markers produced by cardiac marker generator 348, the neural event markers produced by neural marker generator 350, and the physiologic parameters and signals produced by physiologic parameter generator 352.

Zooming input device 466, time range input device 468, timing measurement input device 470, and format input device 472 receive user commands controlling the format of the visual presentation. Zooming input device 466 receives a user selection of a zooming parameter controlling a viewing size of the cardiac and neural signals. Time range input device 468 receives a user selection of a time range associated with the cardiac and neural signals. In one embodiment, time range input device 468 further receives a user command for moving the time range forward or backward in time. Timing measurement input device 470 allows for user-controllable measurement of a time interval between any two points in the cardiac and neural signals. In one embodiment, timing measurement input device 470 includes a caliper controller to control a position of each of two calipers visually displayed with the cardiac and neural signals. The calipers are user-positioned to measure the time interval between any two points in the cardiac and neural signals. In another embodiment, timing measurement input device 470 allows placement of a visually displayed fixed time scale with tick markers and timing labels adjacent to the cardiac and neural signals. In another embodiment, timing measurement input device 470 allows display of the time stamps. In a specific embodiment, the time stamps show absolute times or times relative to a predetermined time reference point. In another specific embodiment, the time stamps show times relative to predetermined type events. Format input device 472 receives a user selection of a visual appearance for each type of the signals to be presented. Examples of such visual appearance include color, gray scale, type of traces (curves), and type of markers (symbols).

Presentation device 426 is a specific embodiment of presentation device 126 and simultaneously presents temporally aligned cardiac and neural signals. In one embodiment, presentation device 426 further presents one or more physiologic parameters or signals simultaneously with the temporally aligned cardiac and neural signals. In one embodiment, presentation device 426 includes a display screen 474, which includes a display area or window for presenting the cardiac and neural signals and/or the physiologic parameters or signals. In another embodiment, presentation device 426 further includes a printer 476. In one specific embodiment, printer 476 starts printing the signals being displayed on display screen 474 on a strip chart upon receiving a user command and stops printing upon receiving another user command.

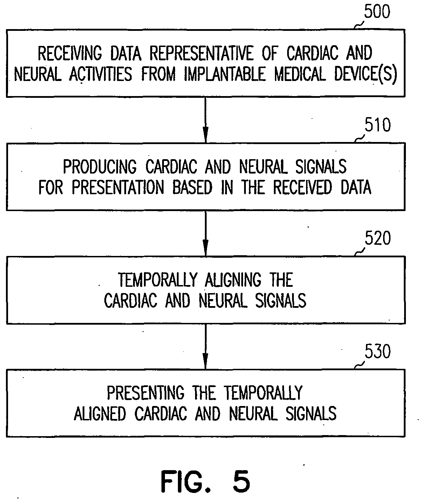

FIG. 5 is a flow chart illustrating an embodiment of a method for simultaneously presenting cardiac and neural signals. In one embodiment, the method is performed using system 100.

Data representative of cardiac and neural activities are received from one or more implantable medical devices at 500. In one embodiment, the data includes timing information indicative times of occurrence for the cardiac and neural activities. In one embodiment, the data represent one or more cardiac signals and one or more neural signals sensed by the one or more implantable medical devices. In another embodiment, the data also represent cardiac event markers representative of cardiac events and/or neural event markers representative of neural events.

Cardiac and neural signals are produced for visual presentation based on the received data at 510. In one embodiment, one or more user commands are received, and cardiac and neural signals are produced according to a user command specifying the types and/or the format of the signals for visual presentation. In one embodiment, a subset of the data representative of cardiac and neural signals associated with a specified period of time is selected according to the user commands specifying that period.

The cardiac and neural signals are temporally aligned at 520. In one embodiment, the cardiac and neural signals are temporally aligned using the timing information received at 500. The temporally aligned cardiac and neural signals are then presented at 530. In one embodiment, the temporally aligned cardiac and neural signals are presented in real time. In another embodiment, the temporally aligned cardiac and neural signals are stored and presented upon receiving a presentation request. The presented cardiac signal(s) include at least one cardiac signal trace and cardiac event markers. The presented neural signal(s) include at least one neural signal trace and neural event markers. The neural event markers include markers indicative of neural stimulation periods each including a time period during which a burst of neural stimulation pulses is delivered. In one embodiment, cardiac event markers, at least one neural signal trace, and neural event markers indicative of the neural stimulation periods are simultaneously displayed. In another embodiment, at least one cardiac signal trace and neural event markers indicative of the neural stimulation periods are simultaneously displayed. In one embodiment, one or more physiologic parameters are measured using the data received from the one or more implantable medical devices and simultaneously displayed with the cardiac and/or neural signals.

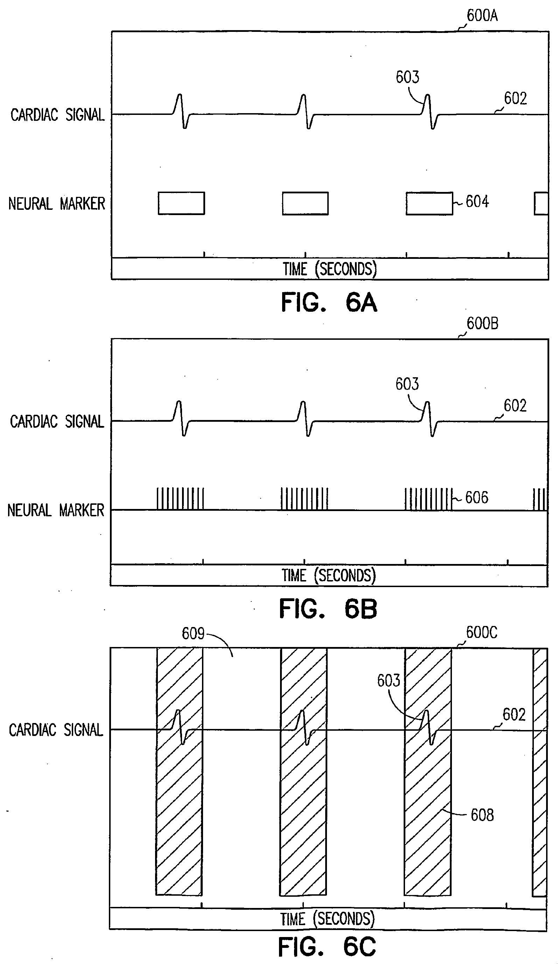

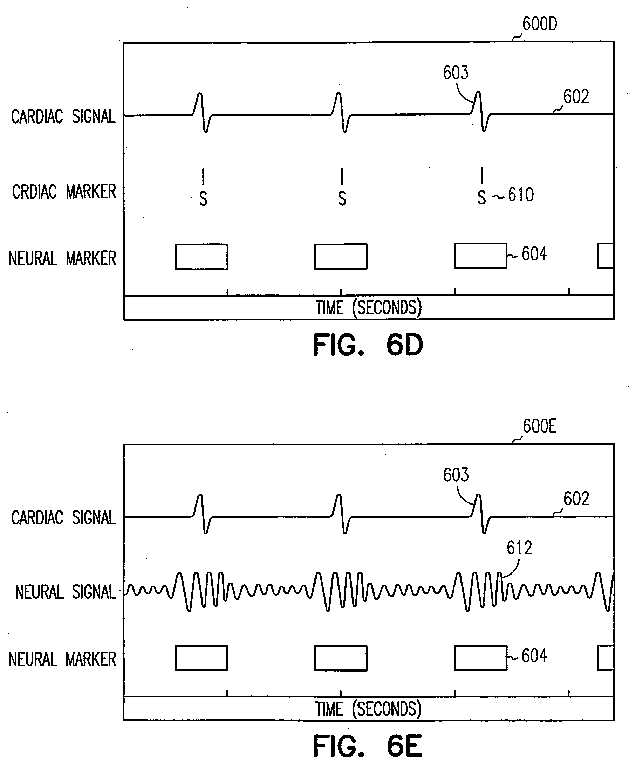

FIGS. 6-10 illustrate various examples of signal presentation according to the present subject matter. These examples are presented for the purpose of illustration but not restriction. According to the present subject matter, both cardiac and neural signals are temporally aligned and simultaneously presented. When available and desirable, one or more physiologic parameters or signals are simultaneously presented with the cardiac and neural signals. Examples of the cardiac signal(s) to be presented include at least one cardiac signal trace and cardiac event markers. The cardiac signal trace is a visual representation of a sensed cardiac signal. The cardiac event markers, or cardiac markers, each present a cardiac event detected from the sensed cardiac signal or a delivery of cardiac stimulation pulse. Examples of the neural signal(s) to be presented include at least one neural signal trace and neural event markers. The neural signal trace is a visual representation of a sensed neural signal. The neural event markers, or neural markers, each present a neural event detected from the sensed neural signal or a delivery of neural stimulation pulse or a neural stimulation period during which a burst of neural stimulation pulses is delivered. In various embodiments, the cardiac and neural markers also include event time information, i.e., the times of occurrence for the events represented by the cardiac and neural markers. In FIGS. 6-10, various specific combinations of signals for simultaneous presentation are illustrated. Other specific combinations are possible, depending on which signals are available for presentation and of interest, as those skilled in the art will understand upon reading and understanding this document. In various embodiments, the specific combination of signals for simultaneous presentation is user-selectable. That is, a physician or other caregiver is allowed to select the types of signals to be simultaneously displayed according to specific diagnostic and/or therapeutic needs. As illustrated in FIGS. 6-10, the presentation device presents the signals on a display screen or a display window being part of the display screen. In various embodiments, the presentation device further includes a printer to print the signals on paper.

FIGS. 6A-E are each an illustration of an exemplary embodiment of a portion of a display screen simultaneously presenting at least a cardiac signal trace and neural event markers. In FIG. 6A, a display window 600A simultaneously displays a cardiac signal trace 602 and neural event markers 604. Cardiac signal trace 602 represents a sensed cardiac signal indicative of cardiac depolarizations 603. As illustrated, neural event markers 604 include rectangular bars each indicative of a neural stimulation period during which a burst of neural stimulation pulses is delivered. In FIG. 6B, a display window 600B simultaneously displays cardiac signal trace 602 and neural event markers 606. As illustrated, neural event markers 606 include symbols each representative of a neural stimulation pulse. In FIG. 6C, a display window 600C simultaneously displays cardiac signal trace 602 and neural event markers 608 and 609. Neural event markers 608 are columns each indicative of a neural stimulation period during which a burst of neural stimulation pulses is delivered. Neural event markers 609 are columns each indicative of a non-stimulation period during which no neural stimulation pulse is delivered. In one embodiment, neural event markers 608 and 609 are displayed in substantially distinctive colors. In another embodiment, neural event markers 608 and 609 are displayed in substantially distinctive gray scales. In another embodiment, neural event markers 608 and 609 displayed with substantially distinctive filling patterns. Neural event markers 606 in FIG. 6B and neural event markers 608 and 609 in FIG. 6C represent exemplary alternatives to neural event markers 604 in FIG. 6A. Any of these types of neural event markers, as well as other symbols having similar visual effects, can be used to indicate the neural stimulation periods. In FIG. 6D, a display window 600D simultaneously displays cardiac signal trace 602, cardiac event markers 610, and neural event markers 604. As illustrated, cardiac event markers 610 include cardiac sense markers each representing one of cardiac depolarizations 603. When cardiac stimulation is delivered, cardiac event markers 610 also include cardiac stimulation markers each representing a delivery of cardiac stimulation pulse. In FIG. 6E, a display window 600E simultaneously displays cardiac signal trace 602, a neural signal trace 612, and neural event markers 604. Neural signal trace 612 represents a sensed neural signal.

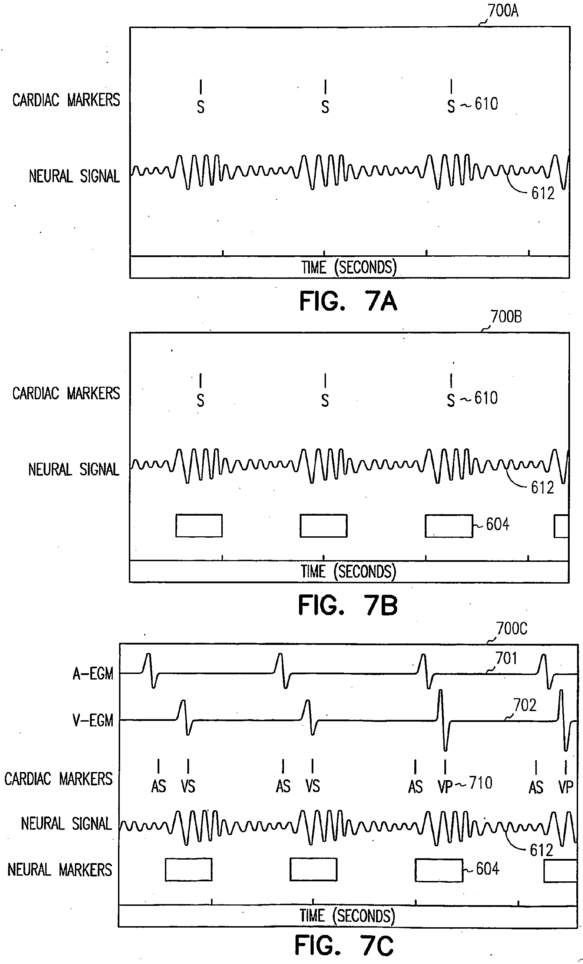

FIG. 7A-C are each an illustration of an exemplary embodiment of a portion of a display screen simultaneously presenting at least a neural signal trace and cardiac event markers. In FIG. 7A, a display window 700A simultaneously displays cardiac event markers 610 and neural signal trace 612. In FIG. 7B, a display window 700B simultaneously displays cardiac event markers 610, neural signal trace 612, and neural event markers 604. In FIG. 7C, a display window 700C simultaneously displays an atrial electrogram (A-EGM) trace 701, a ventricular electrogram (V-EGM) trace 702, cardiac event markers 710, neural signal 612, and neural event markers 604. As illustrated, both cardiac and neural stimulation are applied. A-EGM trace 701 represents a sensed atrial electrogram indicative of atrial depolarizations (P waves). V-EGM trace 702 represents a sensed ventricular electrogram indicative of ventricular depolarizations (R waves) as well as ventricular pacing pulses. Cardiac event markers 710 include cardiac events markers associated with both A-EGM trace 701 and V-EGM trace 702, such as atrial sense markers (As), ventricular sense markers (Vs) and ventricular pace markers (Vp).

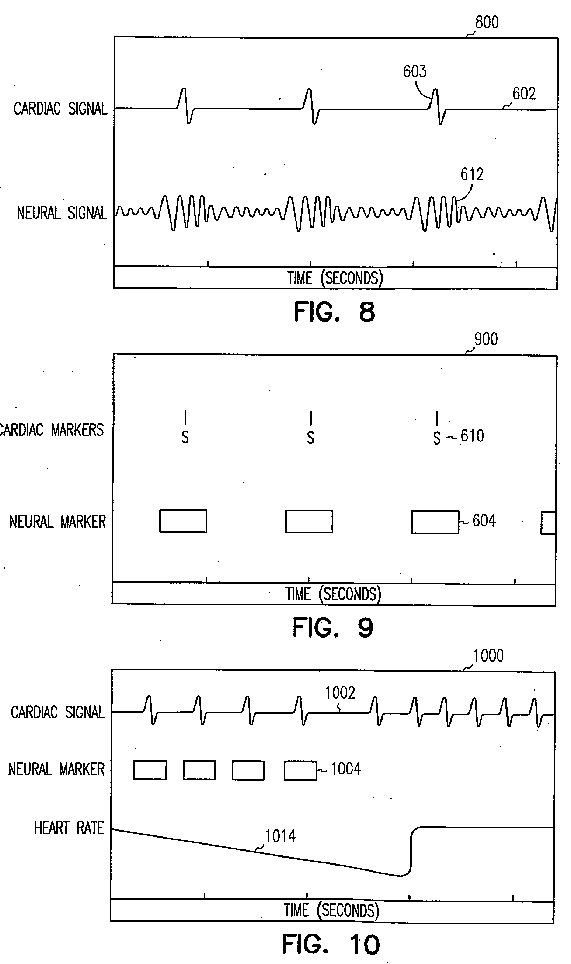

FIG. 8 is an illustration of an exemplary embodiment of a portion of a display screen simultaneously presenting at least a cardiac signal trace and a neural signal trace. A display window 800 simultaneously displays cardiac signal trace 602 and neural signal trace 612.

FIG. 9 is an illustration of an exemplary embodiment of a portion of a display screen presenting at least cardiac event markers and neural event markers. A display window 900 simultaneously displays cardiac event markers 610 and neural event markers 604.

FIG. 10 is an illustration of an exemplary embodiment of a portion of a display screen simultaneously presenting physiologic parameters in addition to the cardiac and neural signals. In FIG. 10, a display window 1000 simultaneously displays a cardiac signal trace 1002, neural event markers 1004, and a physiologic parameter trace 1014. Cardiac signal trace 1002 represents a sensed cardiac signal. Neural event markers 1004 include rectangular bars each indicative of a neural stimulation period during which a burst of neural stimulation pulses is delivered. Physiologic parameter trace 1014 represents a physiologic parameter dynamically derived from the cardiac and/or neural signals. As illustrated in FIG. 10, physiologic parameter trace 1014 represents a heart rate dynamically measured from cardiac signal trace 1002 and shows the effect of neural stimulation on the heart rate.

In various embodiments, in addition to the signal trace(s) and markers illustrated in FIGS. 6-10, a display screen further presents text, numbers, labels, and/or other symbols associated with the signal trace(s) and markers. In various embodiments, a display screen further presents timing information associated with the signal trace(s) and markers, such as a time scale and/or visually displayed time measurement features such as those controllable by the user using timing measurement input device 470.

The simultaneous presentation of cardiac and neural signals provides physicians and other caregivers with a tool used to guide therapy, such as a neural therapy, a cardiac rhythm management (CRM) therapy, or a combined neural and CRM therapy. In various embodiments, the temporally aligned cardiac and neural signals allow monitoring of effects of a neural stimulation therapy in cardiac electrical activities, effects of a cardiac stimulation therapy in neural electrical activities, and/or relations between cardiac and neural activities. Examples of neural signals and their sensing are discussed below to illustrate how system 100, including its various embodiments, is used.

Baroreceptors and chemoreceptors in the heart, great vessels, and lungs transmit cardiac activity through vagal and sympathetic afferent fibers to the central nervous system. Neural leads are used to sense neural signals indicative of neural electrical activities. Various embodiments use a lead placed in a baroreceptor field such as in the aorta, various embodiments use a lead placed in an efferent nerve pathway such as a cardiac fat pad, and various embodiments use a lead placed around a nerve trunk such as the aortic, carotid, and vagus nerves. According to various embodiments, the targeted nerve traffic corresponds to baroreceptors, and thus is useful to determine blood pressure. According to various embodiments, the targeted nerve traffic to be sensed corresponds to chemoreceptors, and thus is useful to determine blood gas concentrations.

A brief discussion of the physiology related to baroreceptors and chemoreceptors is provided below. This brief discussion introduces the autonomic nervous system, baroreflex, and chemoreceptors to provide an understanding of placement of the electrodes (also referred to as neural traffic sensors) of the neural leads and the neural signals sensed using these electrodes.

The autonomic nervous system (ANS) regulates "involuntary" organs, while the contraction of voluntary (skeletal) muscles is controlled by somatic motor nerves. Examples of involuntary organs include respiratory and digestive organs, and also include blood vessels and the heart. Often, the ANS functions in an involuntary, reflexive manner to regulate glands, to regulate muscles in the skin, eye, stomach, intestines and bladder, and to regulate cardiac muscle and the muscle around blood vessels, for example.

The ANS includes, but is not limited to, the sympathetic nervous system and the parasympathetic nervous system. The sympathetic nervous system is affiliated with stress and the "fight or flight response" to emergencies. Among other effects, the "fight or flight response" increases blood pressure and heart rate to increase skeletal muscle blood flow, and decreases digestion to provide the energy for "fighting or fleeing." The parasympathetic nervous system is affiliated with relaxation and the "rest and digest response" which, among other effects, decreases blood pressure and heart rate, and increases digestion to conserve energy. The ANS maintains normal internal function and works with the somatic nervous system.

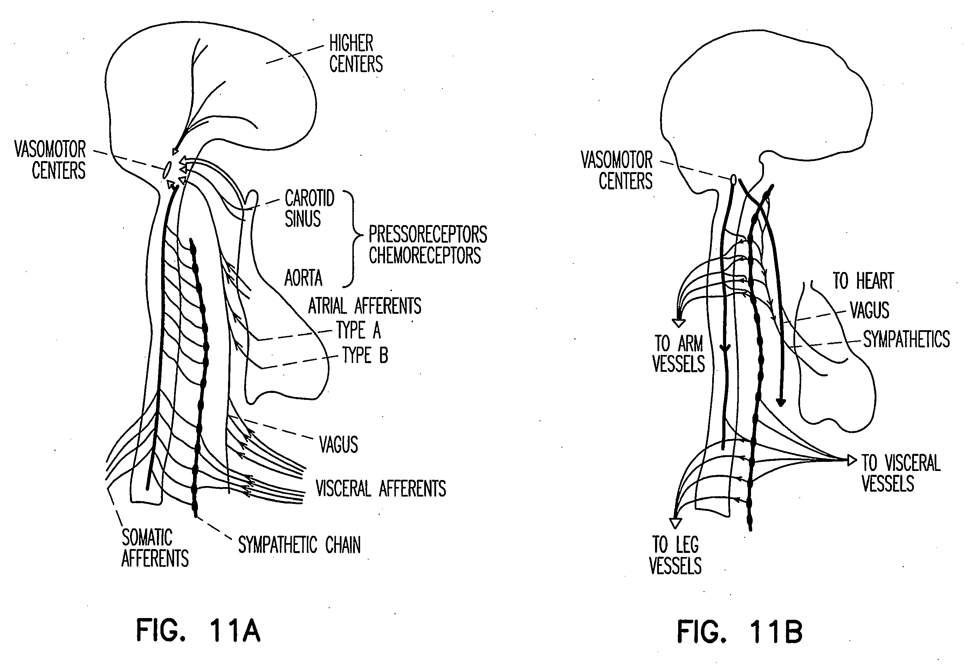

Various embodiments of the present subject matter provide neural stimulation to affect the heart rate, blood pressure, vasodilation and vasoconstriction. The heart rate and force is increased when the sympathetic nervous system is stimulated, and is decreased when the sympathetic nervous system is inhibited (the parasympathetic nervous system is stimulated). Various embodiments detect nerve traffic as a surrogate parameter for another physiologic parameter, such as heart rate, blood pressure and the like. FIGS. 11A and 11B illustrate neural mechanisms for peripheral vascular control. FIG. 11A generally illustrates afferent nerves to vasomotor centers. An afferent nerve conveys impulses toward a nerve center. A vasomotor center relates to nerves that dilate and constrict blood vessels to control the size of the blood vessels. FIG. 11B generally illustrates efferent nerves from vasomotor centers. An efferent nerve conveys impulses away from a nerve center.

Stimulating the sympathetic and parasympathetic nervous systems can have effects other than heart rate and blood pressure. For example, stimulating the sympathetic nervous system dilates the pupil, reduces saliva and mucus production, relaxes the bronchial muscle, reduces the successive waves of involuntary contraction (peristalsis) of the stomach and the motility of the stomach, increases the conversion of glycogen to glucose by the liver, decreases urine secretion by the kidneys, and relaxes the wall and closes the sphincter of the bladder. Stimulating the parasympathetic nervous system and/or inhibiting the sympathetic nervous system constricts the pupil, increases saliva and mucus production, contracts the bronchial muscle, increases secretions and motility in the stomach and large intestine, and increases digestion in the small intention, increases urine secretion, and contracts the wall and relaxes the sphincter of the bladder. The functions associated with the sympathetic and parasympathetic nervous systems are many and can be complexly integrated with each other. Thus, an indiscriminate stimulation of the sympathetic and/or parasympathetic nervous systems to achieve a desired response, such as vasodilation, in one physiological system may also result in an undesired response in other physiological systems. Additionally, sensing of nerve traffic for use as a surrogate parameter of a physiologic parameter can depend on a number of physiologic parameters. Various embodiments of the present subject matter perturb the physiological system with precisely located neural stimulation, and monitor the nerve traffic response to the stimulation.

A pressoreceptive region or field is capable of sensing changes in pressure, such as changes in blood pressure. Pressoreceptor regions are referred to herein as baroreceptors, which generally include any sensors of pressure changes. For example, baroreceptors include afferent nerves and further include sensory nerve endings that provide baroreceptor fields that are sensitive to the stretching of the wall that results from increased blood pressure from within, and function as the receptor of a central reflex mechanism that tends to reduce the pressure. Baroreflex functions as a negative feedback system, and relates to a reflex mechanism triggered by stimulation of a baroreceptor. Increased pressure stretches blood vessels, which in turn activates baroreceptors in the vessel walls. Activation of baroreceptors naturally occurs through internal pressure and stretching of the arterial wall, which excites the parasympathetic nervous system causing baroreflex inhibition of sympathetic nerve activity (SNA) and a reduction in systemic arterial pressure. An increase in baroreceptor activity induces a reduction of SNA, which reduces blood pressure by decreasing peripheral vascular resistance. Centrally mediated reflex pathways modulate cardiac rate, contractility and excitability. Baroreceptors and chemoreceptors in the heart, great vessels, and lungs, transmit neural signals reflective of cardiac activity through vagal and afferent fibers to the central nervous system. Thus, physiologic parameters, such as systemic arterial pressure, can be determined based on nerve traffic. Such pressure information, for example, provides useful feedback information to guide therapy such as neural therapy or CRM therapy such as CRT.

Baroreflex is a reflex triggered by stimulation of a baroreceptor. A baroreceptor includes any sensor of pressure changes, such as sensory nerve endings in the wall of the auricles of the heart, vena cava, aortic arch and carotid sinus, that is sensitive to stretching of the wall resulting from increased pressure from within, and that functions as the receptor of the central reflex mechanism that tends to reduce that pressure. Afferent nerves can also be electrically stimulated to induce a baroreflex, which inhibits the sympathetic nerve activity and stimulates parasympathetic nerve activity. Afferent nerve trunks, such as the vagus, aortic and carotid nerves, leading from the sensory nerve endings also form part of a baroreflex pathway. Stimulating a baroreflex pathway and/or baroreceptors inhibits sympathetic nerve activity, stimulates the parasympathetic nervous system and reduces systemic arterial pressure by decreasing peripheral vascular resistance and cardiac contractility. Baroreceptors are naturally stimulated by internal pressure and the stretching of vessel wall (e.g. arterial wall).

Some aspects of the present subject matter locally sense specific nerve endings in vessel walls rather than or in addition to afferent and/or efferent nerve trunks. For example, some embodiments sense baroreceptor sites or fields in the pulmonary artery. Some embodiments of the present subject matter involve sensing baroreceptor sites or nerve endings in the aorta, the chambers of the heart, some embodiments of the present subject matter involve sensing efferent pathways such as the fat pads of the heart, and some embodiments of the present subject matter involve stimulating an afferent nerve trunk, such as the vagus, carotid and aortic nerves. Various embodiments involve combinations of sensing nerve ending, sensing efferent nerve pathways and sensing afferent nerve pathways. Some embodiments sense nerve trunks using a cuff electrode, and some embodiments sense nerve trunks using an intravascular lead positioned in a blood vessel proximate to the nerve. Examples of afferent nerve trunks include the vagus, aortic and carotid nerves. Examples of efferent nerve trunks include the cardiac branches off the vagus nerve. Stimulation of efferent nerves such as these cardiac branches or the nerves in cardiac fat pads conveys nervous impulses to an effector, and thus do not use the baroreflex negative feedback of the central nervous system, which responds to nerve activity on afferent nerves with nerve activity on efferent nerves. Some embodiments sense neural traffic at any of the above-identified neural stimulation sites.

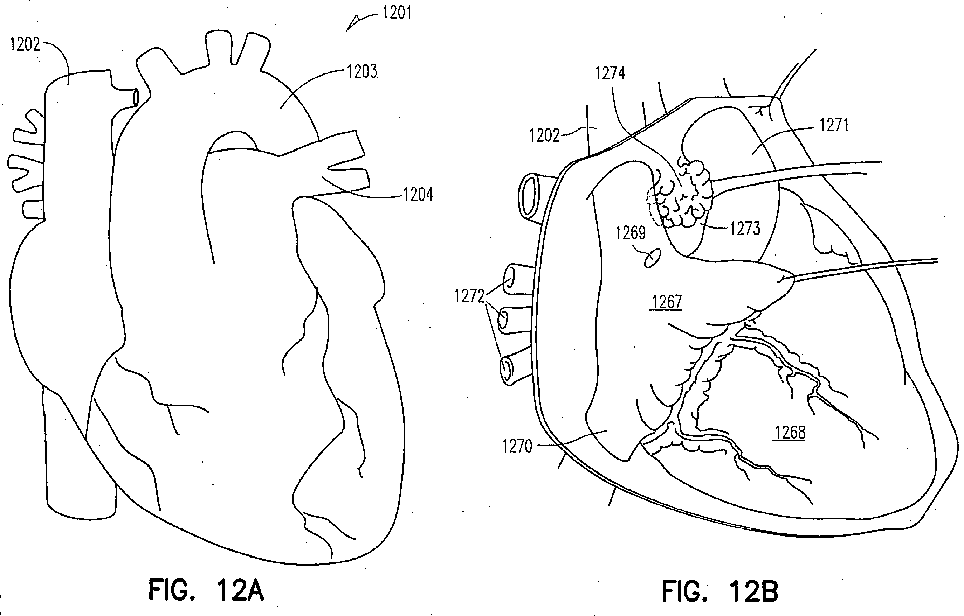

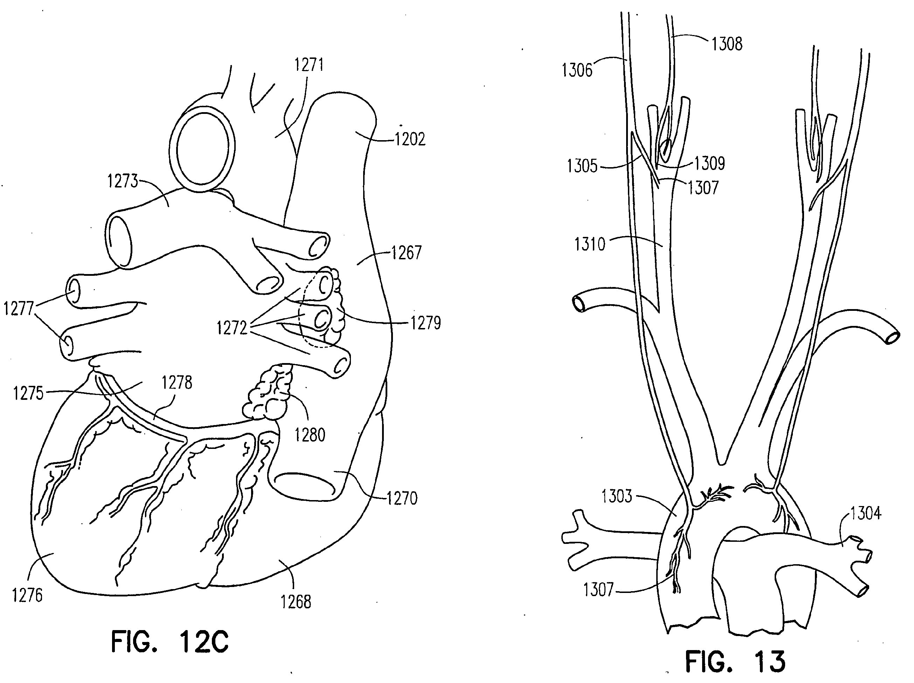

FIGS. 12A-12C illustrate a heart. As illustrated in FIG. 12A, the heart 1201 includes a superior vena cava 1202, an aortic arch 1203, and a pulmonary artery 1204, and is useful to provide a contextual relationship with the illustrations in FIGS. 13-15. As is discussed in more detail below, the pulmonary artery 1204 includes baroreceptors. A lead is capable of being intravascularly inserted through a peripheral vein and through the tricuspid valve into the right ventricle of the heart (not expressly shown in the figure) similar to a cardiac pacemaker lead, and continue from the right ventricle through the pulmonary valve into the pulmonary artery. A portion of the pulmonary artery and aorta are proximate to each other. Various embodiments sense neural activity by the baroreceptor in the aorta using a lead intravascularly positioned in the pulmonary artery. Some embodiments also stimulate baroreceptors in the aorta. Aspects of the present subject matter provide a relatively noninvasive surgical technique to implant a neural traffic sensor, with or without a baroreceptor stimulator, intravascularly into the pulmonary artery.

FIGS. 12B-12C illustrate the right side and left side of the heart, respectively, and further illustrate cardiac fat pads. FIG. 12B illustrates the right atrium 1267, right ventricle 1268, sinoatrial node 1269, superior vena cava 1202, inferior vena cava 1270, aorta 1271, right pulmonary veins 1272, and right pulmonary artery 1273. FIG. 12B also illustrates a cardiac fat pad 1274 between the superior vena cava and aorta. Autonomic ganglia in the cardiac fat pad 1274 are stimulated and/or nerve traffic is sensed in some embodiments using an electrode screwed or otherwise inserted into the fat pad, and are stimulated and/or nerve traffic is sensed in some embodiments using an intravenously-fed lead proximately positioned to the fat pad in a vessel such as the right pulmonary artery or superior vena cava, for example. FIG. 12C illustrates the left atrium 1275, left ventricle 1276, right atrium 1267, right ventricle 1268, superior vena cava 1202, inferior vena cava 1270, aorta 1271, right pulmonary veins 1272, left pulmonary vein 1277, right pulmonary artery 1273, and coronary sinus 1278. FIG. 12C also illustrates a cardiac fat pad 1279 located proximate to the right cardiac veins and a cardiac fat pad 1280 located proximate to the inferior vena cava and left atrium. Autonomic ganglia in the fat pad 1279 are stimulated and/or nerve traffic is sensed in some embodiments using an electrode screwed or otherwise inserted into the fat pad 1279, and are stimulated and/or nerve traffic is sensed in some embodiments using an intravenously-fed lead proximately positioned to the fat pad in a vessel such as the right pulmonary artery 1273 or right pulmonary vein 1272, for example. Autonomic ganglia in the cardiac fat pad 1280 are stimulated and/or nerve traffic is sensed in some embodiments using an electrode screwed or otherwise inserted into the fat pad, and are stimulated and/or nerve traffic is sensed in some embodiments using an intravenously-fed lead proximately positioned to the fat pad in a vessel such as the inferior vena cava 1270 or coronary sinus or a lead in the left atrium 1275, for example.

FIG. 13 illustrates baroreceptors in the area of the carotid sinus 1305, aortic arch 1303 and pulmonary artery 1304. The aortic arch 1303 and pulmonary artery 1304 were previously illustrated with respect to the heart in FIG. 12A. As illustrated in FIG. 13, the vagus nerve 1306 extends and provides sensory nerve endings 1307 that function as baroreceptors in the aortic arch 1303, in the carotid sinus 1305 and in the common carotid artery 1310. The glossopharyngeal nerve 1308 provides nerve endings 1309 that function as baroreceptors in the carotid sinus 1305. These nerve endings 1307 and 1309, for example, are sensitive to stretching of the wall resulting from increased pressure from within. Activation of these nerve endings reduces pressure. Although not illustrated in the figures, the fat pads and the atrial and ventricular chambers of the heart also include baroreceptors. Cuffs have been placed around afferent nerve trunks, such as the vagal nerve, leading from baroreceptors to vasomotor centers to stimulate the baroreflex. According to various embodiments of the present subject matter, afferent nerve trunks can be stimulated and/or nerve traffic from the afferent nerve trunks can be sensed using a cuff or intravascularly-fed lead positioned in a blood vessel proximate to the afferent nerves.

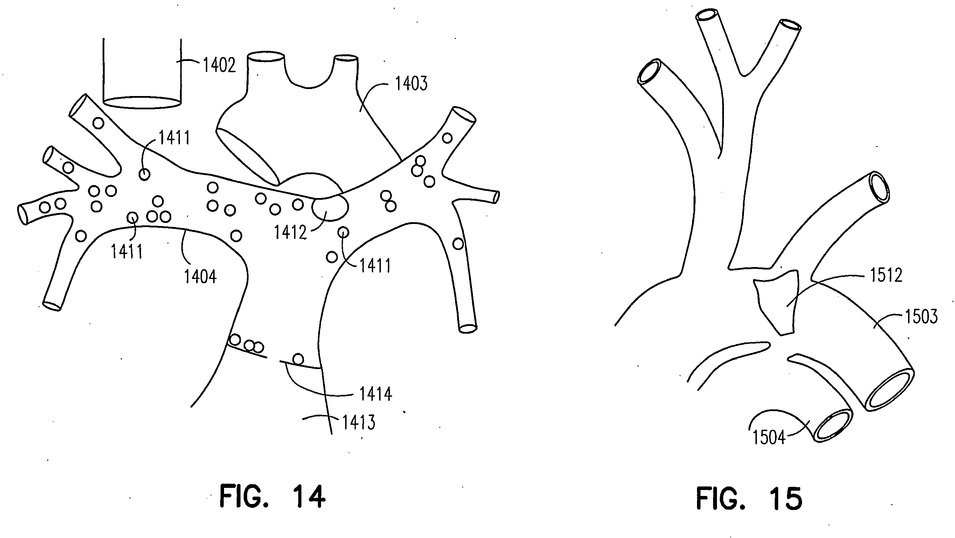

FIG. 14 illustrates baroreceptors in and around a pulmonary artery 1404. The superior vena cava 1402 and the aortic arch 1403 are also illustrated. As illustrated, the pulmonary artery 1404 includes a number of baroreceptors 1411, as generally indicated by the dark area. Furthermore, a cluster of closely spaced baroreceptors is situated near the attachment of the ligamentum arteriosum 1412. FIG. 14 also illustrates the right ventricle 1413 of the heart, and the pulmonary valve 1414 separating the right ventricle 1413 from the pulmonary artery 1404. According to various embodiments of the present subject matter, a lead is inserted through a peripheral vein and threaded through the tricuspid valve into the right ventricle, and from the right ventricle 1413 through the pulmonary valve 1414 and into the pulmonary artery 1404 to stimulate baroreceptors and/or sense nerve traffic from the baroreceptors in and/or around the pulmonary artery. In various embodiments, for example, the lead is positioned to stimulate the cluster of baroreceptors and/or sense nerve traffic near the ligamentum arteriosum 1412.

FIG. 15 illustrates baroreceptor fields 1512 in the aortic arch 1503, near the ligamentum arteriosum and the trunk of the pulmonary artery 1504. Some embodiments position the lead in the pulmonary artery to stimulate baroreceptor sites and/or sense nerve traffic in the aorta and/or fat pads, such as are illustrated in FIGS. 12B-12C.

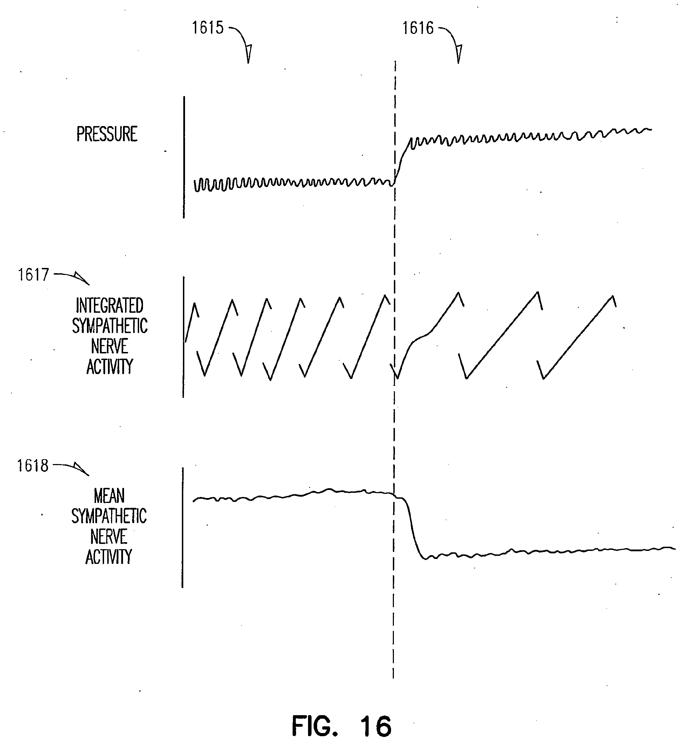

FIG. 16 illustrates an example of a neural response after perturbing a physiologic system. In the illustration, pressure functions as an indicator for a physiologic system. The system is illustrated in a first low pressure condition 1615 and a second high pressure condition 1616. Nerve activity, illustrated at 1617 and 1618, changes between the two conditions. The change may be rather transient in nature if the nervous system quickly adapts from the first to the second condition, or may be more sustained if the nervous system does not quickly adapt to the change in conditions. Regardless, an analysis of a sensed nerve traffic signal can extract or otherwise determine features of the signal indicative of the response. In the illustrated example, the waveform 1617 associated with an integrated sympathetic nerve activity changes (e.g. change in slope and period of waveform) from the first to the second conditions. Additionally, the waveform 1618 associated with a mean sympathetic nerve activity changes (e.g. a first level of nerve activity to a second level of nerve activity) from the first to the second conditions. The integrated sympathetic nerve activity and mean sympathetic nerve activity waveforms are provided as examples. Other ways of sensing changes in the neural traffic signals can be used.

Various embodiments of the present subject matter sense nerve traffic corresponding to chemoreceptors. The carotid and aortic bodies provide a concentration of cardiovascular chemoreceptors. The carotid body lies deep to the bifurcation of the common carotid artery or somewhat between the two branches. The carotid body is a small, flattened, oval structure, 2 to 5 mm in diameter, with a characteristic structure composed of epithelioid cells, which are in close relation to capillary sinusoids, and an abundance of nerve fibers. Surrounding the carotid body is a delicate fibrous capsule. It is part of the visceral afferent system of the body, containing chemoreceptor endings that respond to low levels of oxygen in the blood or high levels of carbon dioxide and lowered pH of the blood. It is supplied by nerve fibers from both the glossopharyngeal and vagus nerves.

The aortic bodies (glomera aortica) are chemoreceptors similar to the carotid bodies. Afferent fibers from the aortic bodies run in the right vagus and have cell bodies in the inferior ganglion. The supracardial bodies (aortic paraganglia) are also chemoreceptors with their afferent fibers in the left vagus and cell bodies in the inferior ganglion.

In various embodiments of the present subject matter, cardiac and neural signals are sensed, and cardiac and neural therapies are delivered, by an implantable system. The implantable system includes an implantable device that has integrated neural stimulation and CRM components or separate implantable neural stimulation and CRM devices. Although implantable systems are illustrated and discussed, various aspects and embodiments of the present subject matter can be implemented in external devices. For example, the cardiac and neural events can be sensed using implantable leads, external electrodes, percutaneous leads, or any combination of these.

FIG. 17 illustrates a cardiac and neural stimulation system 1700, which is a specific embodiment of system 100. System 1700 includes an implantable system 1710 and an external system 1720. Implantable system 1710 is a specific embodiment of implantable system 110 and includes an implantable medical device (IMD) 1780. External system 1720 and IMD 1780 communicates via telemetry link 115. In one embodiment, system 1700 provides for the simultaneous presentation of temporally aligned cardiac and neural signals, and external system 1720 includes presentation device 126 including its specific embodiments.

In various embodiments, IMD 1780 integrates a CRM device with a neural sensing and/or stimulation device. The CRM device senses cardiac electrical activities and delivers cardiac stimulation. Examples of the CRM device include pacemakers, cardioverter/defibrillators, combined pacemaker-cardioverter/defibrillators, cardiac resynchronization therapy (CRT) devices, and cardiac remodeling control therapy (RCT) devices. In various embodiments, neural activities are sensed to indicate a need for cardiac stimulation and/or to control the timing of pacing pulse deliveries. In various embodiments, cardiac activities are sensed to control the timing of neural stimulation pulse deliveries, such as to synchronize neural stimulation to cardiac cycles.

In various embodiments, IMD 1780 includes a sensor to sense ANS activity. In one specific embodiment, the sensed ANS activity provides nerve traffic feedback in a closed loop control system. In various embodiments, surrogate parameters, such as respiration and blood pressure, are sensed to indicate ANS activity. In various embodiments, IMD 1780 delivers neural stimulation to baroreceptors. A neural lead is fed through the right ventricle, and further fed into the pulmonary artery to sense from and/or to deliver neural stimulation pulses to the baroreceptor fields. In various embodiments, neural leads provide access to baroreceptor sites and/or baroreflex pathways, such as those illustrated in FIGS. 12A-12C, 13 and 14, for sensing and/or stimulation.

In one embodiment, implantable system 1710 has a circuit illustrated as the circuit of implantable system 210 in FIG. 2. IMD 1780 is an integrated CRM and neural stimulation device and includes, among other things, a cardiac sensing circuit, a cardiac stimulation circuit, a neural sensing circuit, and a neural stimulation circuit.

FIG. 18 illustrates a cardiac and neural stimulation system 1800, which is another specific embodiment of system 100. System 1800 includes an implantable system 1810 and an external system 1820. Implantable system 1810 is a specific embodiment of implantable system 110 and includes an implantable neural stimulator (NS) device 1882 and an implantable CRM device 1884. External system 1820 and implantable system 1810 communicate via telemetry link 115. In one embodiment, system 1800 provides for the simultaneous presentation of temporally aligned cardiac and neural signals, and external system 1820 includes presentation device 126 including its specific embodiments.

Implantable system 1810 is functionally substantially similar to implantable system 1710 but includes separate CRM and neural stimulation devices. Examples of CRM device 1884 include pacemakers, cardioverter/defibrillators, combined pacemaker-cardioverter/defibrillators, cardiac resynchronization therapy (CRT) devices, and cardiac remodeling control therapy (RCT) devices. NS device 1882 performs the neural sensing and stimulation functions of IMD 1780. A communication link 1885 transmits data representing sensed neural activities and/or neural stimulation activities from NS device 1882 to CRM device 1884, and transmits data representing sensed cardiac activities and/or cardiac stimulation activities from CRM device 1884 to NS device 1882, such that implantable system 1810 can function in a manner substantially similar to implantable system 1710. In one embodiment, communication link 1885 includes a wireless telemetry link using radio-frequency electromagnetic waves or ultrasonic waves as the transmission medium. In another embodiment, communication link 1885 includes one or more leads or cables providing for electrical connections between NS device 1882 and CRM device 1884. In one embodiment, external system 1820 communicates with both NS device 1882 and CRM device 1884 via telemetry link 115. In another embodiment, external system 1820 communicates with one ofNS device 1882 and CRM device 1884 via telemetry link 115, and communicates with the other device further via communication link 1885. In one embodiment, data transmitted from NS device 1882 and CRM device 1884 representing the sensed and stimulation activities in each device are time stamped in a synchronized manner. In a specific embodiment, NS device 1882 and CRM device 1884 exchange time synchronization information to allow use of synchronized clocks in each of the devices for the time stamping. In another embodiment, external system 1820 provides for the time synchronization for the data transmitted from NS device 1882 and CRM device 1884. In a specific embodiment, external system 1820 temporally aligns the signal trace(s) and/or markers to be simultaneously presented by compensating for all known and/or estimated relative time delays associated with transmitting the data from NS device 1882 and CRM device 1884.

In one embodiment, implantable system 1810 has a circuit illustrated as the circuit of implantable system 210 in FIG. 2. The circuit is distributed in NS device 1882 and CRM device 1884. NS device 1882 includes, among other things, a neural sensing circuit and a neural stimulation circuit. CRM device 1884 includes, among other things, a cardiac sensing circuit and a cardiac stimulation circuit.

FIG. 19 is a block diagram illustrating an embodiment of a circuit that provides for the simultaneous presentation of cardiac and neural signals. The circuit is part of a cardiac and neural stimulation system 1900, which is a specific embodiment of system 100. System 1900 includes an implantable system 1910 providing for cardiac and neural sensing and stimulation and an external system 1920.

Implantable system 1910 is a specific embodiment of implantable system 210 and includes leads 1933, a sensing circuit 1935, a stimulation circuit 1937, an implant processing circuit 1942, and an implant telemetry circuit 1944. Leads 1933 include, but are not limited to, various combinations of leads selected from the leads discussed in this document. Sensing circuit 1935 senses cardiac and neural signals through leads 1933. Stimulation circuit 1937 delivers cardiac and/or neural stimulation pulses through leads 1933. Implant processing circuit 1942, which is part of implant control circuit 242, produces data representative of the sensed cardiac and neural signals and deliveries of the cardiac and/or neural stimulation pulses. In one embodiment, implant processing circuit 1942 generates cardiac and neural event markers to represent cardiac and neural events including both sensed activities and the deliveries of the cardiac and neural stimulation pulses. Implant telemetry circuit 1944 transmits the data to external system 1920.

External system 1920 is a specific embodiment of external system 120 and includes an external telemetry circuit 1922, an external processing circuit 1924, and a presentation device 1926. External telemetry circuit 1922 receives the data from implantable system 1910. External processing circuit 1924, which is part of external control circuit 124 including presentation controller 128, processes the received data to produce and temporally align cardiac and neural signals for simultaneous presentation by presentation device 1926.

Implant processing circuit 1942 and external processing circuit 1924 form a signal processing circuit 1946, which produces the cardiac and neural signals for presentation based on the sensed cardiac and neural signals. Signal processing circuit 1946 illustrates that signal processing circuit 346 is distributed in an implantable system and an external system according to one embodiment of the present subject matter.

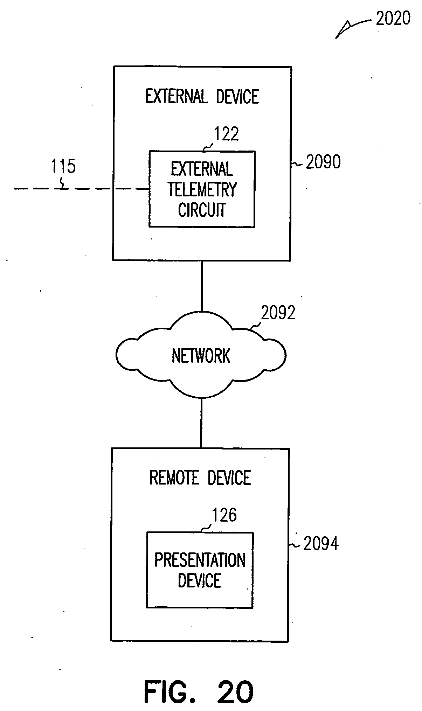

FIG. 20 is a block diagram illustrating a specific embodiment of an external system 2020, which is a specific embodiment of external system 120, 1720, 1820, or 1920. As illustrated in FIG. 20, external system 2020 is a patient management system including an external device 2090, a telecommunication network 2092, and a remote device 2094. External device 2090 is placed within the vicinity of an implantable system and includes external telemetry system 122 to communicate with the implantable system via telemetry link 115. Remote device 2094 is in a remote location and communicates with external device 2090 through network 2092, thus allowing a physician or other caregiver to monitor and treat a patient from a distant location and/or allowing access to various treatment resources from the remote location. Remote device 2094 includes presentation device 126.

It is to be understood that the above detailed description is intended to be illustrative, and not restrictive. Other embodiments will be apparent to those of skill in the art upon reading and understanding the above description. The scope of the invention should, therefore, be determined with reference to the appended claims, along with the full scope of legal equivalents to which such claims are entitled.

## Claims

1. A system (120) for presenting signals sensed by one or more implantable medical devices (110, 1710, 1810), the system comprising:
a telemetry circuit (122, 1922) configured to receive data representative of time stamped cardiac and neural activities transmitted from the one or more implantable medical devices, the received data including one or more intracardiac electrograms indicative of cardiac depolarizations, one or more sensed nerve traffic signals indicative of autonomic nerve traffic, and neural event markers indicative of neural stimulation periods each including a time period during which a burst of neural stimulation pulses is delivered;
an external control circuit (124) coupled to the telemetry circuit, the external control circuit including a presentation controller (128) adapted to produce and temporally align one or more cardiac signals (602, 610, 701, 702, 710, 1002) indicative of cardiac depolarizations and neural signals (604, 606, 608, 609, 612, 1004) indicative of the autonomic nerve traffic and the neural stimulation periods for visual presentation based on the received data including time stamps for the cardiac and neural activities; and
a presentation device (126,426, 1926) coupled to the presentation controller, the presentation device adapted to simultaneously present the temporally aligned one or more cardiac signals and the neural signals to allow for observation of relationships between the cardiac and neural activities.

2. The system according to claim 1, wherein the presentation device comprises a display screen (474).

3. The system according to any of the preceding claims, wherein the external control circuit further comprises a physiologic parameter generator (352) adapted to derive one or more physiologic parameters from the received data, and wherein the presentation device is further adapted to display the one or more physiologic parameters (1014) simultaneously with the one or more cardiac signals and one or more neural signals.

4. The system according to any of the preceding claims, further comprising a user input (425) to receive one or more user commands, and wherein the presentation controller is adapted to produce and temporally align the one or more cardiac signals and one or more neural signals according to the one or more user commands.

5. The system according to claim 4, wherein the user input comprises a time range input (468) device adapted to receive a user selection of a time range associated with the one or more cardiac signals and one or more neural signals.

6. The system according to any of the preceding claims, wherein the one or more cardiac signals comprise one or more of at least one cardiac signal trace (602. 701, 702, 1002) representing a sensed cardiac signal and cardiac event markers (610, 710) each representative of a predetermined type cardiac event, and the one or more neural signals comprise one or more of at least one neural signal trace (612) representing a sensed neural signal and neural event markers (604, 606, 608, 609, 1004) each representative of a predetermined type neural event, wherein the sensed cardiac and neural signals are sensed by the one or more implantable medical devices.

7. The system according to claim 6, wherein the neural event markers comprise neural event markers each indicative of a neural stimulation period during which a burst of neural stimulation pulses is delivered.

8. The system according to any of claims 6 and 7, wherein the presentation device is adapted to present the at least one cardiac signal trace and the neural event markers simultaneously.

9. The system according to any of claims 6 and 7, wherein the presentation device is adapted to present the at least one neural signal trace and the cardiac event markers simultaneously.

10. The system according to claim 6, wherein the presentation device is adapted to present the at least one cardiac signal trace and the at least one neural signal trace simultaneously.

11. The system according to any of claims 6 and 7, wherein the presentation device is adapted to present the cardiac event markers and the neural event markers simultaneously.

12. The system according to any of the preceding claims further comprising the one or more implantable medical devices, and wherein the one or more implantable medical devices comprise:
a cardiac sensing circuit (232) to sense the cardiac activities;
a cardiac stimulation circuit (234) to deliver cardiac stimulation pulses;
a neural sensing circuit (238) to sense the neural activities;
a neural stimulation circuit (240) to deliver neural stimulation pulses;
an implant control circuit (242), coupled to the cardiac sensing circuit, the cardiac stimulation circuit, the neural sensing circuit, and the neural stimulation circuit, to produce the data representative of the cardiac and neural activities including the delivered cardiac stimulation pulses and the delivered neural stimulation pulses; and
an implant telemetry circuit (244), coupled to the implant control circuit, to transmit the data representative of the cardiac and neural activities, and wherein the one or more cardiac signals are representative of at least one of the cardiac activities and the delivered cardiac stimulation pulses, and the one or more neural signals are representative of at least one of the neural electrical activities and the delivered neural stimulation pulses.

13. The system according to claim 12, wherein the one or more implantable medical devices comprise an implantable medical device (1780) including at least the cardiac sensing circuit, the cardiac stimulation circuit, the neural sensing circuit, and the neural stimulation circuit.

14. The system according to claim 12, wherein the one or more implantable medical devices comprise:
an implantable cardiac rhythm management device (1884) including at least the cardiac sensing circuit and the cardiac stimulation circuit; and
an implantable neural stimulation device (1882) including at least the neural sensing circuit and the neural stimulation circuit.

15. A method, comprising:
receiving data representative of time stamped cardiac and neural activities from one or more implantable medical devices, the received data including one or more intracardiac electrograms indicative of cardiac depolarizations, one or more sensed nerve traffic signals indicative of autonomic nerve traffic, and neural event markers indicative of neural stimulation periods each including a time period during which a burst of neural stimulation pulses is delivered;
producing cardiac signals indicative of cardiac depolarizations and neural signals indicative of the autonomic nerve traffic and the neural stimulation periods for visual presentation based on the received data including time stamps for the cardiac and neural activities;
aligning the cardiac and neural signals temporally; and
simultaneously presenting the temporally aligned one or more cardiac signals and the neural signals to allow for observation of relationships between the cardiac and neural activities.

16. The method according to claim 15, further comprising:
receiving one or more user commands; and
producing cardiac and neural signals for presentation according to the one or more user commands.

17. The method according to claim 16, wherein receiving the one or more user commands comprises receiving a user command selecting a subset of the data representative of cardiac and neural activities occurring or detected during a specified period of time.

18. The method according to any of claims 15 to 17, further comprising:
deriving one or more physiologic parameters from the received data; and
presenting the one or more physiologic parameters simultaneously with the temporally aligned cardiac and neural signals.

19. The method according to any of claims 15 to 18, wherein presenting the temporally aligned cardiac and neural signals comprises presenting the temporally aligned cardiac and neural signals in real time.

20. The method according to any of claims 15 to 19, further comprising:
storing the received data; and
receiving a presentation request, wherein presenting the temporally aligned cardiac and neural signals comprises presenting the temporally aligned cardiac and neural signals in response to the presentation request.

21. The method according to any of claims 15 to 20, wherein presenting the temporally aligned cardiac and neural signals comprises:
displaying one or more of at least one cardiac signal trace and cardiac event markers;
and
displaying one or more of at least one neural signal trace and neural event markers.

22. The method according to any of claims 15 to 21, wherein presenting the temporally aligned cardiac and neural signals comprises presenting neural event markers indicative of neural stimulation periods each including a time period during which a burst of neural stimulation pulses is delivered.

23. The method according to claim 22, wherein presenting the temporally aligned cardiac and neural signals comprises:
displaying the cardiac event markers;
displaying the at least one neural signal; and
displaying the neural event markers including the neural event markers indicative of the neural stimulation periods.

24. The method according to claim 22, wherein presenting the temporally aligned cardiac and neural signals comprises:
displaying the at least one cardiac signal; and
displaying the neural event markers including the neural event markers indicative of the neural stimulation periods.

## Patentansprüche

1. System (120) zum Darstellen von Signalen, die durch eine oder mehr implantierbare medizinische Vorrichtungen (110, 1710, 1810) erfasst wurden, welches System aufweist:
eine Telemetrieschaltung (122, 1922), die konfiguriert ist zum Empfangen von Daten, die repräsentativ für zeitgestempelte Herz- und Nervenaktivitäten, die von der einen oder den mehreren implantierbaren medizinischen Vorrichtungen gesendet wurden, sind, wobei die empfangenen Daten ein oder mehrere intrakardiale Elektrogramme, die Herzdepolarisationen anzeigen, ein oder mehrere erfasste Nervenverkehrssignale, die vegetativen Nervenverkehr anzeigen, und Nervenereignismarkierer, die Nervenstimulationsperioden anzeigen, die jeweils eine Zeitperiode enthalten, während der ein Bündel von Nervenstimulationsimpulsen geliefert wird, enthalten;
eine externe Steuerschaltung (124), die mit der Telemetrieschaltung gekoppelt ist, wobei die externe Steuerschaltung eine Darstellungssteuervorrichtung (128) enthält, die ausgestaltet ist zum Erzeugen und zeitlichen Ausrichten eines oder mehrerer Herzsignale (602, 610, 701, 702, 710, 1002), die Herzdepolarisationen anzeigen, und Nervensignale (604, 606, 608, 609, 612, 1004), die den vegetativen Nervenverkehr und die Nervenstimulationsperioden anzeigen, für visuelle Darstellung auf der Grundlage der empfangenen Daten enthaltend Zeitstempel für die Herz- und Nervenaktivitäten; und
eine Darstellungsvorrichtung (126, 426, 1926), die mit der Darstellungssteuervorrichtung gekoppelt ist, wobei die Darstellungsvorrichtung ausgestaltet ist zum gleichzeitigen Darstellen der zeitlich ausgerichteten einen oder mehreren Herzsignale und der Nervensignale, um eine Beobachtung der Beziehungen zwischen den Herz- und Nervenaktivitäten zu ermöglichen.

2. System nach Anspruch 1, bei dem die Darstellungsvorrichtung einen Anzeigeschirm (474) aufweist.

3. System nach einem der vorhergehenden Ansprüche, bei dem die externe Steuerschaltung weiterhin einen Generator (352) für physiologische Parameter aufweist, der ausgestaltet ist zum Ableiten eines oder mehrerer physiologischer Parameter aus den empfangenen Daten, und bei dem die Darstellungsvorrichtung weiterhin ausgestaltet ist zum Anzeigen des einen oder der mehreren physiologischen Parameter (1014) gleichzeitig mit dem einen oder den mehreren Herzsignalen und dem einen oder den mehreren Nervensignalen.

4. System nach einem der vorhergehenden Ansprüche, weiterhin aufweisend einen Benutzereingang (425) zum Empfangen von einem oder mehreren Benutzerbefehlen, und bei dem die Darstellungssteuervorrichtung ausgestaltet ist zum Erzeugen und zeitlichen Ausrichten des einen oder der mehreren Herzsignale und des einen oder der mehreren Nervensignale gemäß dem einen oder den mehreren Benutzerbefehlen.

5. System nach Anspruch 4, bei dem der Benutzereingang eine Zeitbereichs-Eingangsvorrichtung (468) aufweist, die ausgestaltet ist zum Empfangen einer Benutzerauswahl eines Zeitbereichs, der mit dem einen oder den mehreren Herzsignalen und dem einen oder den mehreren Nervensignalen assoziiert ist.

6. System nach einem der vorhergehenden Ansprüche, bei dem das eine oder die mehreren Herzsignale eine oder mehrere von zumindest einer Herzsignalkurve (602, 701, 702, 1002), die ein erfasstes Herzsignal und Herzereignismarkierer (610, 710), die jeweils repräsentativ für einen vorbestimmten Herzereignistyp sind, aufweisen, und die einen oder mehreren Nervensignale eine oder mehrere von zumindest einer Nervensignalkurve (612), die ein erfasstes Nervensignal und Nervenereignismarkierer (604, 606, 608, 609, 1004) darstellen, die jeweils repräsentativ für einen vorbestimmten Nervenereignistyp sind, aufweisen, wobei die erfassten Herz- und Nervensignale durch die eine oder die mehreren implantierbaren medizinischen Vorrichtungen erfasst werden.

7. System nach Anspruch 6, bei dem die Nervenereignismarkierer solche Nervenereignismarkierer aufweisen, die jeweils eine Nervenstimulationsperiode anzeigen, während der ein Bündel von Nervenstimulationsimpulsen geliefert wird.

8. System nach einem der Ansprüche 6 und 7, bei dem die Darstellungsvorrichtung ausgestaltet ist zum gleichzeitigen Darstellen der zumindest einen Herzsignalkurve und der Nervenereignismarkierer.

9. System nach einem der Ansprüche 6 und 7, bei dem die Darstellungsvorrichtung ausgestaltet ist zum gleichzeitigen Darstellen der zumindest einen Nervensignalkurve und der Herzereignismarkierer.

10. System nach Anspruch 6, bei dem die Darstellungsvorrichtung ausgestaltet ist zum gleichzeitigen Darstellen der zumindest einen Herzsignalkurve und der zumindest einen Nervensignalkurve.

11. System nach einem der Ansprüche 6 und 7, bei dem die Darstellungsvorrichtung ausgestaltet ist zum gleichzeitigen Darstellen der Herzereignismarkierer und der Nervenereignismarkierer.

12. System nach einem der vorhergehenden Ansprüche, weiterhin aufweisend die eine oder die mehreren implantierbaren medizinischen Vorrichtungen, und bei dem die eine oder die mehreren implantierbaren medizinischen Vorrichtungen aufweisen:
eine Herzerfassungsschaltung (232) zum Erfassen der Herzaktivitäten; eine Herzstimulationsschaltung (234) zum Liefern von Herzstimulationsimpulsen;
eine Nervenerfassungsschaltung (238) zum Erfassen der Nervenaktivitäten;
eine Nervenstimulationsschaltung (240) zum Liefern von Nervenstimulationsimpulsen;
eine Implantatsteuerschaltung (242), die mit der Herzerfassungsschaltung, der Herzstimulationsschaltung, der Nervenerfassungsschaltung und der Nervenstimulationsschaltung gekoppelt ist, um die Daten zu erzeugen, die repräsentativ für die Herz- und Nervenaktivitäten enthaltend die gelieferten Herzstimulationsimpulse und die gelieferten Nervenstimulationsimpulse sind; und
eine Implantattelemetrieschaltung (244), die mit der Implantatsteuerschaltung gekoppelt ist, um die Daten, die für die Herz- und Nervenaktivitäten repräsentativ sind, zu senden, und wobei das eine oder die mehreren Herzsignale repräsentativ sind für zumindest eine der Herzaktivitäten und der gelieferten Herzstimulationsimpulse, und dass eine oder mehrere Nervensignale repräsentativ sind für zumindest eine der elektrischen Nervenaktivitäten und die gelieferten Nervenstimulationsimpulse.

13. System nach Anspruch 12, bei dem die eine oder die mehreren implantierbaren medizinischen Vorrichtungen eine implantierbare medizinische Vorrichtung (1780) aufweisen, die zumindest die Herzerfassungsschaltung, die Herzstimulationsschaltung, die Nervenerfassungsschaltung und die Nervenstimulationsschaltung enthält.

14. System nach Anspruch 12, bei dem die eine oder die mehreren implantierbaren medizinischen Vorrichtungen aufweisen:
eine implantierbare Herzrhythmus-Managementvorrichtung (1884) enthaltend zumindest die Herzerfassungsschaltung und die Herzstimulationsschaltung; und
eine implantierbare Nervenstimulationsvorrichtung (1882) enthaltend zumindest die Nervenerfassungsschaltung und die Nervenstimulationsschaltung.

15. Verfahren, welches aufweist:
Empfangen von Daten, die repräsentativ sind für zeitgestempelte Herzund Nervenaktivitäten, von einer oder mehreren implantierbaren medizinischen Vorrichtungen, wobei die empfangenen Daten ein oder
mehrere intrakardiale Elektrogramme, die Herzdepolarisationen anzeigen, ein oder mehrere erfasste Nervenverkehrssignale, die einen vegetativen Nervenverkehr anzeigen, und Nervenereignismarkierer, die Nervenstimulationsperioden anzeigen, die jeweils eine Zeitperiode enthalten, während der ein Bündel von Nervenstimulationsimpulsen geliefert wird, enthalten;
Erzeugen von Herzsignalen, die Herzdepolarisationen anzeigen, und
Nervensignalen, die den vegetativen Nervenverkehr und die Nervenstimulationsperioden für visuelle Darstellung auf der Grundlage der empfangenen Daten enthaltend Zeitstempel für die Herz- und Nervenaktivitäten anzeigen;
zeitliches Ausrichten der Herz- und Nervensignale; und
gleichzeitiges Darstellen der zeitlich ausgerichteten einen oder mehreren Herzsignale und der Nervensignale, um eine Beobachtung von Beziehungen zwischen den Herz- und Nervenaktivitäten zu ermöglichen.

16. Verfahren nach Anspruch 15, welches weiterhin aufweist:
Empfangen von einem oder mehreren Benutzerbefehlen; und
Erzeugen von Herz- und Nervensignalen für eine Darstellung gemäß dem einen oder den mehreren Benutzerbefehlen.

17. Verfahren nach Anspruch 16, bei dem das Empfangen des einen oder der mehreren Benutzerbefehle das Empfangen eines Benutzerbefehls, der einen Subsatz der Daten, die repräsentativ für Herz- und Nervenaktivitäten sind, die während einer bestimmten Zeitperiode auftreten oder erfasst werden, auswählt, aufweist.

18. Verfahren nach einem der Ansprüche 15 bis 17, welches weiterhin aufweist:
Ableiten eines oder mehrerer physiologischer Parameter aus den empfangenen Daten; und
gleichzeitiges Darstellen des einen oder der mehreren physiologischen Parameter mit den zeitlich ausgerichteten Herz- und Nervensignalen.

19. Verfahren nach einem der Ansprüche 15 bis 18, bei dem das Darstellen der zeitlich ausgerichteten Herz- und Nervensignale das Darstellen der zeitlich ausgerichteten Herz- und Nervensignale in Echtzeit aufweist.

20. Verfahren nach einem der Ansprüche 15 bis 19, welches weiterhin aufweist:
Speichern der empfangenen Daten; und
Empfangen einer Darstellungsanforderung, wobei das Darstellen der zeitlich ausgerichteten Herz- und Nervensignale das Darstellen der zeitlich ausgerichteten Herz- und Nervensignale als Antwort auf die Darstellungsanforderung aufweist.

21. Verfahren nach einem der Ansprüche 15 bis 20, bei dem das Darstellen der zeitlich ausgerichteten Herz- und Nervensignale aufweist:
Anzeigen von einer oder mehreren von zumindest einer Herzsignalkurve und Herzereignismarkierern; und
Anzeigen von einer oder mehreren von zumindest einer Nervensignalkurve und Nervenereignismarkierern.

22. Verfahren nach einem der Ansprüche 15 bis 21, bei dem das Darstellen der zeitlich ausgerichteten Herz- und Nervensignale das Darstellen von Nervenereignismarkierern, die Nervenstimulationsperioden anzeigen, die jeweils eine Zeitperiode enthalten, während der ein Bündel von Nervenstimulationsimpulsen geliefert wird, aufweist.

23. Verfahren nach Anspruch 22, bei dem das Darstellen der zeitlich ausgerichteten Herz- und Nervensignale aufweist:
Anzeigen der Herzereignismarkierer;
Anzeigen des zumindest einen Nervensignals; und
Anzeigen der Nervenereignismarkierer enthaltend die Nervenereignismarkierer, die für die Nervenstimulationsperioden anzeigend sind.

24. Verfahren nach Anspruch 22, bei dem das Darstellen der zeitlich ausgerichteten Herz- und Nervensignale aufweist:
Anzeigen des zumindest einen Herzsignals; und
Anzeigen der Nervenereignismarkierer enthalten die Nervenereignismarkierer, die für die Nervenstimulationsperiode anzeigend sind.

## Revendications

1. Système (120) pour présenter des signaux détectés par un ou plusieurs dispositif(s) médical/médicaux implantable(s) (110, 1710, 1810), le système comprenant :
un circuit de télémétrie (122, 1922) configuré de manière à recevoir des données représentatives d'activités cardiaques et neurales timbrées temporellement qui sont transmises depuis les un ou plusieurs dispositif(s) médical/médicaux implantable(s), les données reçues incluant un ou plusieurs électrocardiogramme(s) intracardiaque(s) indicatif(s) de dépolarisations cardiaques, un ou plusieurs signal/signaux de trafic nerveux détecté(s) indicatif(s) d'un trafic nerveux autonome et des marqueurs d'événement neural indicatifs de périodes de stimulation neurale dont chacune inclut une période temporelle pendant laquelle une salve d'impulsions de stimulation neurale est délivrée ;
un circuit de commande externe (124) couplé au circuit de télémétrie, le circuit de commande externe incluant un contrôleur de présentation (128) adapté de manière à produire et à aligner temporellement un ou plusieurs signal/signaux cardiaque(s) (602, 610, 701, 702, 710, 1002) indicatif(s) de dépolarisations cardiaques et des signaux neuraux (604, 606, 608, 609, 612, 1004) indicatifs du trafic nerveux autonome et les périodes de stimulation neurale pour une présentation visuelle sur la base des données reçues incluant des timbres temporels pour les activités cardiaques et neurales ; et
un dispositif de présentation (126, 426, 1926) couplé au contrôleur de présentation, le dispositif de présentation étant adapté de manière à présenter simultanément les un ou plusieurs signal/signaux cardiaque(s) aligné(s) temporellement et les signaux neuraux afin de permettre l'observation de relations entre les activités cardiaques et neurales.

2. Système selon la revendication 1, dans lequel le dispositif de présentation comprend un écran d'affichage (474).

3. Système selon l'une quelconque des revendications précédentes, dans lequel le circuit de commande externe comprend en outre un générateur de paramètre physiologique (352) adapté de manière à dériver un ou plusieurs paramètre(s) physiologique(s) à partir des données reçues, et dans lequel le dispositif de présentation est en outre adapté de manière à afficher les un ou plusieurs paramètre(s) physiologique(s) (1014) simultanément à l'affichage des un ou plusieurs signal/signaux cardiaque(s) et des un ou plusieurs signal/signaux neural/neuraux.

4. Système selon l'une quelconque des revendications précédentes, comprenant en outre une entrée utilisateur (425) pour recevoir une ou plusieurs commande(s) d'utilisateur, et dans lequel le contrôleur de présentation est adapté de manière à produire et à aligner temporellement les un ou plusieurs signal/signaux cardiaque(s) et les un ou plusieurs signal/signaux neuraux conformément aux une ou plusieurs commande(s) d'utilisateur.

5. Système selon la revendication 4, dans lequel l'entrée utilisateur comprend un dispositif d'entrée de plage temporelle (468) adapté de manière à recevoir une sélection par utilisateur d'une plage temporelle associée au(x) un ou plusieurs signal/signaux cardiaque(s) et au(x) un ou plusieurs signal/signaux neuraux.

6. Système selon l'une quelconque des revendications précédentes, dans lequel les un ou plusieurs signal/signaux cardiaque(s) comprennent un ou plusieurs tracé(s) pris parmi au moins un tracé de signal cardiaque (602, 701, 702, 1002) représentant un signal cardiaque détecté et des marqueurs d'événement cardiaque (610, 710) dont chacun est représentatif d'un événement cardiaque de type prédéterminé, et les un ou plusieurs signal/signaux neural/neuraux comprennent un ou plusieurs tracé(s) pris parmi au moins un tracé de signal neural (612) représentant un signal neural détecté et des marqueurs d'événement neural (604, 606, 608, 609, 1004) dont chacun est représentatif d'un événement neural de type prédéterminé, dans lequel les signaux cardiaques et neuraux détectés sont détectés par les un ou plusieurs dispositif(s) médical/médicaux implantable(s).

7. Système selon la revendication 6, dans lequel les marqueurs d'événement neural comprennent des marqueurs d'événement neural dont chacun est indicatif d'une période de stimulation neurale pendant laquelle une salve d'impulsions de stimulation neurale est délivrée.

8. Système selon l'une quelconque des revendications 6 et 7, dans lequel le dispositif de présentation est adapté de manière à présenter l'au moins un tracé de signal cardiaque et les marqueurs d'événement neural simultanément.

9. Système selon l'une quelconque des revendications 6 et 7, dans lequel le dispositif de présentation est adapté de manière à présenter l'au moins un tracé de signal neural et les marqueurs d'événement cardiaque simultanément.

10. Système selon la revendication 6, dans lequel le dispositif de présentation est adapté de manière à présenter l'au moins un tracé de signal cardiaque et l'au moins un tracé de signal neural simultanément.

11. Système selon l'une quelconque des revendications 6 et 7, dans lequel le dispositif de présentation est adapté de manière à présenter les marqueurs d'événement cardiaque et les marqueurs d'événement neural simultanément.

12. Système selon l'une quelconque des revendications précédentes, comprenant en outre les un ou plusieurs dispositif(s) médical/médicaux implantable(s), dans lequel les un ou plusieurs dispositif(s) médical/médicaux implantable(s) comprennent :
un circuit de détection cardiaque (232) pour détecter les activités cardiaques ;
un circuit de stimulation cardiaque (234) pour délivrer des impulsions de stimulation cardiaque ;
un circuit de détection neurale (238) pour détecter les activités neurales ;
un circuit de stimulation neurale (240) pour délivrer des impulsions de stimulation neurale ;
un circuit de commande d'implant (242), couplé au circuit de détection cardiaque, au circuit de stimulation cardiaque, au circuit de détection neurale et au circuit de stimulation neurale afin de produire les données représentatives des activités cardiaques et neurales incluant les impulsions de stimulation cardiaque délivrées et les impulsions de stimulation neurale délivrées ; et
un circuit de télémétrie d'implant (244), couplé au circuit de commande d'implant, afin de transmettre les données représentatives des activités cardiaques et neurales, et dans lequel les un ou plusieurs signal/signaux cardiaque(s) sont représentatifs d'au moins l'une des activités cardiaques et des impulsions de stimulation cardiaque délivrées et les un ou plusieurs signal/signaux neural/neuraux sont représentatifs d'au moins l'une des activités électriques neurales et des impulsions de stimulation neurale délivrées.

13. Système selon la revendication 12, dans lequel les un ou plusieurs dispositif(s) médical/médicaux implantable(s) comprennent un dispositif médical implantable (1780) incluant au moins le circuit de détection cardiaque, le circuit de stimulation cardiaque, le circuit de détection neurale et le circuit de stimulation neurale.

14. Système selon la revendication 12, dans lequel les un ou plusieurs dispositif(s) médical/médicaux implantable(s) comprennent :
un dispositif de gestion de rythme cardiaque implantable (1884) incluant au moins le circuit de détection cardiaque et le circuit de stimulation cardiaque ; et
un dispositif de stimulation neurale implantable (1882) incluant au moins le circuit de détection neurale et le circuit de stimulation neurale.

15. Procédé, comprenant :
la réception de données représentatives d'activités cardiaques et neurales timbrées temporellement en provenance d'un ou de plusieurs dispositif(s) médical/médicaux implantable(s), les données reçues incluant un ou plusieurs électrocardiogramme(s) intracardiaque(s) indicatif(s) de dépolarisations cardiaques, un ou plusieurs signal/signaux de trafic nerveux détecté(s) indicatif(s) d'un trafic nerveux autonome et des marqueurs d'événement neural indicatifs de périodes de stimulation neurale dont chacune inclut une période temporelle pendant laquelle une salve d'impulsions de stimulation neurale est délivrée ;
la production de signaux cardiaques indicatifs de dépolarisations cardiaques et de signaux neuraux indicatifs du trafic nerveux autonome et des périodes de stimulation neurale pour une présentation visuelle sur la base des données reçues incluant des timbres temporels pour les activités cardiaques et neurales ;
l'alignement des signaux cardiaques et neuraux temporellement ; et
la présentation de façon simultanée des un ou plusieurs signal/signaux cardiaque(s) aligné(s) temporellement et des signaux neuraux afin de permettre l'observation de relations entre les activités cardiaques et neurales.

16. Procédé selon la revendication 15, comprenant en outre :
la réception d'une ou de plusieurs commande(s) d'utilisateur ; et
la production de signaux cardiaques et neuraux pour une présentation conformément aux une ou plusieurs commande(s) d'utilisateur.

17. Procédé selon la revendication 16, dans lequel la réception des une ou plusieurs commande(s) d'utilisateur comprend la réception d'une commande d'utilisateur sélectionnant un sous-jeu des données représentatives d'activités cardiaques et neurales survenant ou détectées pendant une période temporelle spécifiée.

18. Procédé selon l'une quelconque des revendications 15 à 17, comprenant en outre :
la dérivation d'un ou de plusieurs paramètre(s) physiologique(s) à partir des données reçues ; et
la présentation des un ou plusieurs paramètre(s) physiologique(s) simultanément à la présentation des signaux cardiaques et neuraux alignés temporellement.

19. Procédé selon l'une quelconque des revendications 15 à 18, dans lequel la présentation des signaux cardiaques et neuraux alignés temporellement comprend la présentation des signaux cardiaques et neuraux alignés temporellement en temps réel.

20. Procédé selon l'une quelconque des revendications 15 à 19, comprenant en outre :
le stockage des données reçues ; et
la réception d'une requête de présentation, dans lequel la présentation des signaux cardiaques et neuraux alignés temporellement comprend la présentation des signaux cardiaques et neuraux alignés temporellement en réponse à la requête de présentation.

21. Procédé selon l'une quelconque des revendications 15 à 20, dans lequel la présentation des signaux cardiaques et neuraux alignés temporellement comprend :
l'affichage d'un ou de plusieurs d'au moins un tracé de signal cardiaque et de marqueurs d'événement cardiaque ; et
l'affichage d'un ou de plusieurs d'au moins un tracé de signal neural et de marqueurs d'événement neural.

22. Procédé selon l'une quelconque des revendications 15 à 21, dans lequel la présentation des signaux cardiaques et neuraux alignés temporellement comprend la présentation de marqueurs d'événement neural indicatifs de périodes de stimulation neurale dont chacune inclut une période temporelle pendant laquelle une salve d'impulsions de stimulation neurale est délivrée.

23. Procédé selon la revendication 22, dans lequel la présentation des signaux cardiaques et neuraux alignés temporellement comprend :
l'affichage des marqueurs d'événement cardiaque ;
l'affichage de l'au moins un signal neural ; et
l'affichage des marqueurs d'événement neural incluant les marqueurs d'événement neural indicatifs des périodes de stimulation neurale.

24. Procédé selon la revendication 22, dans lequel la présentation des signaux cardiaques et neuraux alignés temporellement comprend :
l'affichage de l'au moins un signal cardiaque ; et
l'affichage des marqueurs d'événement neural incluant les marqueurs d'événement neural indicatifs des périodes de stimulation neurale.
